# EUROPEAN PATENT APPLICATION

(11) **EP 3 213 753 A1**
(43) Date of publication of application: **06.09.2017**
(21) Application number: 15854921.2
(22) Date of filing: 23.07.2015
(51) Int. Cl.: A61K 31/7034, A61K 8/60, A61P 17/16, A61P 43/00, A61Q 19/00

(54) **SKIN BARRIER FUNCTION-IMPROVING AGENT AND COMPOSITION FOR IMPROVING SKIN BARRIER FUNCTION**

(30) Priority: 29.10.2014 JP 2014220711
(71) Applicant: Showa Denko K.K., Tokyo 105-8518 (JP)
(72) Inventor: SAEKI, Yuko, Tokyo 105-8518 (JP); KATO, Eiko, Tokyo 105-8518 (JP)
(74) Representative: Strehl Schübel-Hopf & Partner
(86) International application number: PCT/JP2015/070915
(87) International publication number: WO 2016/067688

(57) **Abstract**

The present invention provides a skin barrier function-improving agent including an inositol derivative as an active ingredient in which inositol and saccharide are bonded, and a composition for improving a skin barrier function including the above skin barrier function-improving agent and a pharmacologically acceptable carrier.

## Description

### Technical Field

The present invention relates to a skin barrier function-improving agent and a composition for improving a skin barrier function. Priority is claimed on Japanese Patent Application No. 2014-220711, filed on October 29, 2014, the content of which is incorporated herein by reference.

### Background Art

Transient receptor potential vanilloid 4 (TRPV4) is a protein expressed as an osmoreceptor which is activated by low osmotic pressure.

It is reported that TRPV4 has a function as a water loss sensor, and by being activated by 4α-phorbol ester (4α-PDD), TRPV4 reduces moisture loss and enhances a skin barrier function (for example, refer to Non-Patent Document 1 and Non-Patent Document 2).

The skin barrier function is closely related to tight junctions (TJ). TJ is a belt-shaped intercellular adhesion structure that is formed around an epithelial cell. TJs seal intercellular spaces by closely linking adjacent epithelial cells to each other, and therefore entering and leaving of substances through the spaces are prevented. TJs are developed in the skin, trachea, intestinal tract, blood vessels, or the like, and perform a barrier function such that the passage of fluids inside tissues and entry of foreign substances from outside are prevented. As membrane proteins localized in TJs, claudins, occludin, or the like are known. It is known that claudins include 24 members. Among members, claudin-1 is responsible for forming TJ strands and plays an important role in forming an intercellular barrier of the epidermis.

It is reported that activation of TRPV4 present in the skin promotes TJ formation, which leads to the improvement of a skin barrier function, and therefore is effective on improving rough skin and skin moisture (for example, refer to Non-Patent Document 3 and Patent Document 1).

### Citation List

### Patent Literature

[Patent Document 1] Japanese Unexamined Patent Application, First Publication No. 2011-93830

### Non-Patent Literature

[Non-Patent Document 1] Denda M., et al., Effects of Skin Surface Temperature on Epidermal Permeability Barrier Homeostasis, J. Invest. Dermatol., 127(3), 654 to 659, 2007.
[Non-Patent Document 2] Denda M., Methodology to improve epidermal barrier homeostasis: How to accelerate the barrier recovery?, International Journal of Cosmetic Science, 31, 79 to 86, 2009.
[Non-Patent Document 3] Kida N., et al., Importance of transient receptor potential vanilloid 4 (TRPV4) in epidermal barrier function in human skin keratinocytes, Pflugers Arch., 463(5), 715 to 725, 2012.

### Summary of Invention

### Technical Problem

There are cases where the skin barrier function-improving agent of the related art does not have a sufficient ability to improve a moisture-retaining function of the skin. Here, an object of the present invention is to provide a skin barrier function-improving agent and a composition for improving a skin barrier function which act on both TRPV4 and TJs and effectively improve the moisture-retaining function of the skin.

### Solution to Problem

The present invention is as follows.
(1) A skin barrier function-improving agent including an inositol derivative as an active ingredient in which inositol and saccharide are bonded.
(2) The skin barrier function-improving agent according to (1), in which the saccharide is monosaccharide or oligosaccharide.
(3) The skin barrier function-improving agent according to (2), in which the monosaccharide is glucose.
(4) The skin barrier function-improving agent according to (2), in which the oligosaccharide contains glucose as a structural unit.
(5) The skin barrier function-improving agent according to any one of (1) to (4), in which the inositol is myo-inositol.
(6) The skin barrier function-improving agent according to any one of (1) to (5), which promotes the production of TRPV4.
(7) The skin barrier function-improving agent according to any one of (1) to (6), which promotes the production of claudins.
(8) The skin barrier function-improving agent according to any one of (1) to (7), which promotes the production of occludin.
(9) A composition for improving a skin barrier function including the skin barrier function-improving agent according to any one of (1) to (8), and a pharmacologically acceptable carrier.
(10) The composition for improving a skin barrier function according to (9), in which the amount of the skin barrier function-improving agent is 0.01 to 50% by mass.
(11) The composition for improving a skin barrier function according to (9) or (10), which is a skin external agent.
(12) The composition for improving a skin barrier function according to any one of (9) to (11), which is a cosmetic.

### Advantageous Effects of Invention

According to the present invention, it is possible to provide a skin barrier function-improving agent and a composition for improving a skin barrier function which act on both TRPV4 and TJs and effectively improve a moisture-retaining function of the skin.

### Description of Embodiments

### [Skin Barrier Function-improving Agent]

In one embodiment, the present invention provides a skin barrier function-improving agent including an inositol derivative as an active ingredient in which inositol and saccharide are bonded.

As described below, the skin barrier function-improving agent of the embodiment is effective on promoting the production of TRPV4, claudins, and occludin, and is very safe even when administered into a living body. By promoting the production of TRPV4, claudins, and occludin, the formation of tight junctions is effectively promoted, and thus a moisture-retaining function of the skin is effectively enhanced. Accordingly, it can be said that the skin barrier function-improving agent of the embodiment is a promoter for TRPV4 production. In addition, it can be said that the skin barrier function-improving agent of the embodiment is a promoter for claudins production. Furthermore, it can be said that the skin barrier function-improving agent of the embodiment is a promoter for occludin production. Furthermore, it can be said that the skin barrier function-improving agent of the embodiment is an enhancer for the moisture-retaining function of the skin. Furthermore, enhancing the moisture-retaining function of the skin leads to the improvement of xerosis cutis. Therefore, it can be said that the skin barrier function-improving agent of the embodiment is a therapeutic agent for xerosis cutis.

### (Inositol Derivative)

The skin barrier function-improving agent of the embodiment includes an inositol derivative as an active ingredient in which inositol and saccharide are bonded.

Inositol is a cyclic hexahydric alcohol represented by C₆H₆(OH)₆. Inositol exists in nine stereoisomers which are cis-inositol, epi-inositol, allo-inositol, myo-inositol, muco-inositol, neo-inositol, chiro-inositol (D-chiro-inositol and L-chiro-inositol are present), and scyllo-inositol.

In the skin barrier function-improving agent of the embodiment, it is preferable that inositol constituting an inositol derivative is myo-inositol which is the only bioactive isomer among the above isomers. Inositol can be synthesized by a rice bran extraction method, a chemical synthesis, a fermentation method, or the like.

In the skin barrier function-improving agent of the embodiment, an inositol derivative is a compound in which saccharide is bonded to a hydroxyl group of inositol. Saccharide may be bonded to any one or two or more of six hydroxyl groups present in inositol molecules. For example, as an inositol derivative, an inositol derivative in which saccharide is bonded to any one of six hydroxyl groups of inositol molecules may be used. In addition, in an inositol derivative, a plurality of inositol molecules may be bonded to one saccharide molecule.

Saccharide constituting an inositol derivative is not particularly limited, and examples thereof include mannitol, sorbitol, xylitol, maltitol, erythritol, pentaerythritol, glucose, sucrose, fructose, lactose, maltose, xylose, trehalose, α-cyclodextrin, β-cyclodextrin, γ-cyclodextrin, and the like. Saccharide constituting an inositol derivative may be monosaccharide or oligosaccharide. Saccharide constituting an inositol derivative may be glucose or oligosaccharide containing glucose as a structural unit. The above oligosaccharide may contain only glucose as a structural unit.

Alternatively, the above oligosaccharide may contain at least one glucose molecule and saccharide other than glucose as a structural unit. The molecular weight of the above oligosaccharide may be, for example, approximately 300 to 3,000. Specific examples of oligosaccharide include disaccharides such as sucrose, lactose, maltose, trehalose and cellobiose; trisaccharides such as raffinose, melezitose, and maltotriose; and tetrasaccharides such as stachyose.

In addition, an inositol derivative may be a mixture of an inositol derivative in which saccharides are monosaccharides, and an inositol derivative in which saccharides are oligosaccharides.

From the point of view of easily obtaining an inositol derivative having a high degree of purification, it is preferable to use β-cyclodextrin which is industrially inexpensive and stably suppliable as a raw material of an inositol derivative. In this case, saccharide constituting an inositol derivative contains glucose as a structural unit. On the other hand, if starch or the like, which is cheaper, is used as a raw material of an inositol derivative, various kinds of saccharides are transferred to various sites when synthesizing an inositol derivative, and thus, a degree of purification of an inositol derivative obtained tends to be unstable.

### (Method for Synthesizing Inositol Derivative)

A method for synthesizing an inositol derivative is not particularly limited, and an inositol derivative can be appropriately synthesized by the known method of the related art. For example, an inositol derivative may be synthesized by reacting inositol with cyclodextrin which is a type of oligosaccharide in the presence of cyclodextrin glucanotransferase (refer to, for example, Japanese Unexamined Patent Application, First Publication No. S63-196596). Alternatively, an inositol derivative may be synthesized by a method in which a glucosylated inositol derivative is obtained by using glucosyl phosphite as a saccharide donor (refer to, for example, Japanese Unexamined Patent Application, First Publication No. H6-298783).

The skin barrier function-improving agent of the embodiment as it is may be administered orally or parenterally, or may be administered orally or parenterally in the form of a composition for improving a skin barrier function mixed with a suitable pharmacologically acceptable carrier.

### [Composition for Improving Skin Barrier Function]

In one embodiment, the present invention provides a composition for improving a skin barrier function including the above skin barrier function-improving agent and a pharmacologically acceptable carrier.

The above skin barrier function-improving agent can be administered orally in the form of, for example, a tablet, a coated tablet, a pill, a powder, a granule, a capsule, a solution, a suspension, or an emulsion or can be administered parenterally in the form of an injection, a suppository, or a skin external agent according to a commonly used method, as the composition for improving a skin barrier function in which the pharmacologically acceptable carrier is mixed.

The above skin external agent may be a cosmetic. Examples of the kinds of skin external agent or cosmetic include a hair cosmetic selected from a shampoo, an oil shampoo, a cream shampoo, a conditioning shampoo, a shampoo for dandruff, a shampoo for hair color, a rinse integrated shampoo, a rinse, a treatment, a hair pack, a hair foam, a hair mousse, a hair spray, a hair mist, a hair wax, a hair gel, a water grease, a setting lotion, a color lotion, a hair tonic, a hair liquid, a pomade, a chick, a hair cream, a hair blow, a split hair coater, a hair oil, an agent for permanent wave, a straight permanent treatment agent, an oxidation hair dye, a hair bleach, a hair color pretreatment, a hair color after treatment, a permanent pretreatment, a permanent after treatment, a hair manicure, and a hair growth tonic; a basic cosmetic selected from a facial cleanser, a cleansing foam, a soap powder, a facial cleansing powder, a cleansing cream, a cleansing milk, a cleansing lotion, a cleansing gel, a cleansing oil, a cleansing mask, a lotion, a softening lotion, an astringent lotion, a lotion for cleansing, a multilayered lotion, an emulsion, an emollient lotion, a moisture lotion, a milky lotion, a nourishing lotion, a nourishing milk, a skin moisturizer, a moisture emulsion, a massage lotion, a cleansing lotion, a protection emulsion, sun protection, a sun protector, a UV care milk, a sun screen, a makeup lotion, a keratin smoother, an elbow lotion, a hand lotion, a body lotion, a cream, an emollient cream, a nutritive cream, a nourishing cream, a vanishing cream, a moisture cream, a night cream, a massage cream, a cleansing cream, a makeup cream, a base cream, a pre-makeup cream, a sunscreen cream, a suntan cream, a hair removal cream, a deodorant cream, a shaving cream, a keratin layer softening cream, a gel, a cleansing gel, a moisture gel, a soap, a toilet soap, a transparent soap, a medicated soap, a liquid soap, a shaving soap, a synthetic toilet soap, a pack, a mask, a peel off pack, a powder pack, a washing pack, an oil pack, a cleansing mask, an essence, a moisturizing essence, a whitening essence, a UV protection essence, a liposome essence, and a liposome lotion; a makeup cosmetic selected from a face powder, dusting powder, foundations, a makeup base, lipsticks, a lip gloss, rouges, an eyeliner, a mascara, an eye shadow, an eyebrow pencil, an eyebrow, a nail enamel, an enamel remover, and a nail treatment; a fragrant cosmetic selected from a perfume, a perfume, a parfum, eau de parfum, eau de toilette, eau de cologne, a solid perfume, an aromatic powder, a perfume soap, a body lotion, and a bath oil; and body cosmetics selected from a body shampoo, a body cleanser, a body powder, a deodorant lotion, a deodorant powder, a deodorant spray, a deodorant stick, deodorant cosmetics, a bleaching agent, a depilation-epilation agent, a bath agent, a bug repellent spray, and an insect repeller; an ointment, a patch, a lotion, a liniment, and a liquid coating agent.

Examples of the dosage form of a skin external agent or a cosmetic include emulsion forms such as an oil-in-water (O/W) form, a water-in-oil (W/O) form, a W/O/W form, and an O/W/O form, an emulsion polymer form, an oily form, a solid form, a liquid form, a paste form, a stick form, a volatile oil form, a powder form, a jelly form, a gel form, a paste form, a cream form, a sheet form, a film form, a mist form, a spray form, a multi-layered form, a foam form, and a flake form.

The composition for improving a skin barrier function of the embodiment may be a health food. Examples of dosage forms of the health food include a tablet, a coated tablet, a pill, a powder, a granule, a capsule, a solution, a suspension, an emulsion, and the like.

The composition for improving a skin barrier function of the embodiment may contain a raw material described in existing raw material specifications or official compendiums in a general concentration, for example, 100 ppm to 90% by mass with respect to the total amount of the composition for improving a skin barrier function. As the above raw material, for example, all raw materials described in the Japanese Pharmacopoeia, 14th edition (edited by Pharmaceutical and Medical Device Regulatory Science Society of Japan, published by Jiho Inc., in April 2001), Japanese Standards of Cosmetic Ingredients, 2nd edition commentary (edited by Pharmaceutical and Medical Device Regulatory Science Society of Japan, published by Yakuji Nippo Ltd., 1984), Supplement to The Japanese Cosmetic Ingredients Codex (Ministry of Health and Welfare Pharmaceutical Affairs Bureau examination Division supervision, published by Yakuji Nippo Ltd., 1993), Supplement to The Japanese Cosmetic Ingredients Codex, Supplement (Ministry of Health and Welfare Pharmaceutical Affairs Bureau examination Division supervision, published by Yakuji Nippo Ltd., 1993), The Comprehensive Licensing Standards Of Cosmetics by Category (Ministry of Health and Welfare Pharmaceutical Affairs Bureau examination Division supervision, Yakuji Nippo Ltd., 1993), International Cosmetic Ingredient Dictionary and Handbook 2002, Ninth Edition Vol. 1-4, by CTFA, and Cosmetic Ingredients Dictionary (published by Nikko Chemicals, 1991) can be used.

As the pharmacologically acceptable carrier, for example, one or more kinds selected from an excipient, a binding agent, a disintegrating agent, a lubricant, an emulsifier, a stabilizer, a diluent, a solvent for injection, an oily base, a moisturizing agent, a touch improving agent, a surfactant, a polymer, a thickening-gelling agent, a solvent, a propellant, an antioxidant, a reductant, an oxidant, a chelating agent, an acid, an alkali, a powder, an inorganic salt, water, a metal-containing compound, an unsaturated monomer, polyhydric alcohol, a polymer additive, an auxiliary agent, a wetting agent, a thickening agent, a tackifying material, an oily material, a liquid matrix, fat-soluble material, and polymer carboxylate can be used.

The composition for improving a skin barrier function of the embodiment may further contain one or more kinds selected from a preservative, an antibacterial agent, an ultraviolet absorbent, a whitening agent, vitamins and derivatives thereof, an antiphlogistic agent, an antiinflammatory agent, a drug for hair growth, a blood circulation accelerator, a stimulant, hormone, an antiwrinkle agent, an antiaging agent, a tightening agent, a cold sense agent, a warm sense agent, a wound healing accelerator, an irritation alleviating agent, an analgesic, a cell activator, plant, animal, and microbial extract, an antipruritic agent, a keratin releasing-dissolving agent, an antiperspirant, a cooling agent, an astringent, enzyme, nucleic acid, fragrance, coloring matter, a colorant, a dye, a pigment, an antiinflammatory analgesic agent, an antifungal agent, an antihistamine, a hypnotic sedative, a tranquilizer, an antihypertensive agent, a hypotensive diuretic, an antibiotic, an anesthetic, an antibacterial substance, an antiepileptic agent, a coronary vasodilator, an herbal medicine, an antipruritic agent, a keratin layer softening and peeling agent, an ultraviolet screening agent, an antiseptic preservative, an antioxidant, a pH adjusting agent, an additive, and a metal soap.

Examples of the excipient include an organic excipient and an inorganic excipient. Examples of the organic excipient include sugar derivatives such as lactose and white sugar; starch derivatives such as corn starch and potato starch; cellulose derivatives such as crystalline cellulose; and gum arabic. As the inorganic excipient, one or more kinds selected from sulfates such as calcium sulfate can be used.

As the binding agent, for example, one or more kinds selected from the above-described excipients, gelatin, polyvinyl pyrrolidone, and polyethylene glycol can be used.

Examples of the disintegrating agent, for example, one or more kinds selected from the above-described excipients; derivatives of starch or cellulose such as croscarmellose sodium, and carboxymethyl starch sodium; and crosslinked polyvinyl pyrrolidone can be used.

As the lubricant, for example, one or more kinds selected from talc; stearic acid; colloidal silica; waxes such as beeswax and spermaceti; sulfates such as sodium sulfate; laurylsulfates such as sodium laurylsulfate; and starch derivatives among the above-described excipients can be used.

As the emulsifier, for example, one or more kinds selected from colloidal clays such as bentonite and veegum; anionic surfactants such as sodium laurylsulfate; cationic surfactants such as benzalkonium chloride; nonionic surfactants such as polyoxyethylene alkyl ether; and (poly)glyceryl fatty acid ester surfactants such as polyglyceryl stearate-10, polyglyceryl distearate-10, polyglyceryl tristearate-10, and polyglyceryl pentastearate-10 can be used.

As the stabilizer, for example, one or more kinds selected from parahydroxybenzoic acid esters such as methyl paraben and propyl paraben; alcohols such as chlorobutanol; and phenols such as phenol and cresol can be used.

As the diluent, for example, one or more kinds selected from water, ethanol, and propylene glycol can be used.

As the solvent for injection, for example, one or more kinds selected from water, ethanol, and glycerine can be used.

As the oily base, for example, one or more kinds selected from higher alcohols such as cetanol, myristyl alcohol, oleyl alcohol, lauryl alcohol, cetostearyl alcohol, stearyl alcohol, arachyl alcohol, behenyl alcohol, jojoba alcohol, chimyl alcohol, selachyl alcohol, batyl alcohol, hexyldecanol, isostearyl alcohol, 2-octyl dodecanol, and a dimer diol; aralkyl alcohols such as benzyl alcohol and derivatives thereof; higher fatty acids such as lauric acid, myristic acid, palmitic acid, stearic acid, isostearic acid, behenic acid, undecylenic acid, 12-hydroxystearic acid, palmitoleic acid, oleic acid, linoleic acid, linoleic acid, erucic acid, docosahexaenoic acid, eicosapentaenoic acid, isohexadecanoic acid, anteheneicosanoic acid, a long-chain branched fatty acid, a dimer acid, and a hydrogenated dimer acid, and aluminum salts thereof; metal soaps such as a calcium salt, a magnesium salt, a zinc salt, and a potassium salt, and nitrogen-containing derivatives such as amide; hydrocarbons such as liquid paraffin (mineral oil), heavy liquid isoparaffin, light liquid isoparaffin, an α-olefin oligomer, polyisobutene, hydrogenated polyisobutene, polybutene, squalane, olive-derived squalane, squalene, vaseline, and solid paraffin; waxes such as candelilla wax, carnauba wax, rice wax, Japan wax, beeswax, montan wax, ozokerite, ceresine, paraffin wax, microcrystalline wax, petrolatum, Fischer-Tropsch wax, polyethylene wax, and an ethylene-propylene copolymer; vegetable fats and oils such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao oil, shea oil, hydrogenated coconut oil, hydrogenated castor oil, jojoba oil, and hydrogenated jojoba oil; animal fats and oils such as beef tallow, milk fat, horse fat, egg yolk oil, mink oil, and turtle oil; animal waxes such as spermacetieti, lanolin, and orange roughy oil; lanolins such as liquid lanolin, reduced lanolin, adsorption-purified lanolin, lanolin acetate, liquid lanolin acetate, hydroxy lanolin, polyoxyethylene lanolin, lanolin fatty acid, hard lanoline fatty acid, lanolin alcohol, acetylated lanolin alcohol, and acetic acid (cetyl/lanolyl) ester; phospholipids such as lecithin, phosphatidylcholine, phosphatidylethanolamine, phosphatidylserine, phosphatidylglycerol, phosphatidylinositol, sphingophospholipids such as sphingomyelin, phosphatidic acid, and lysolecithin; phospholipid derivatives such as hydrogenated soybean phospholipid, partially hydrogenated soybean phospholipid, hydrogenated egg yolk phospholipid, and partially hydrogenated egg yolk phospholipid; sterols such as cholesterol, dihydrocholesterol, lanosterol, dihydrolanosterol, phytosterol, and cholic acid; sapogenins; saponins; sterol esters such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acylsarcosine alkyl esters such as isopropyl N-lauroyl sarcosinate, cholesteryl 12-hydroxystearate, cholesteryl macadamiate, phytosteryl macadamiate, phytosteryl isostearate, soft cholesteryl lanolin, hard cholesteryl lanolin, long-chain branched fatty acid cholesterol, and long-chain α-hydroxyfatty acid cholesterol; lipid complexes such as a phospholipid-cholesterol complex and a phospholipid-phytosterol complex; monoalcohol-carboxylic acid esters such as octyldodecyl myristate, hexyldecyl myristate, octyldodecyl isostearate, cetyl palmitate, octyl dodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyl octanoate, octyldodecyl erucate, hard castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate; oxyacid esters such as cetyl lactate, diisostearyl malate, and hydrogenated castor oil monoisostearate; polyhydric alcohol fatty acid esters such as glyceryl trioctanoate, glyceryl trioleate, glyceryl triisostearate, glyceryl diisostearate, triglyceride (caprylate/capriate), triglyceryl (caprylate/caprinate/myristate/stearate), hydrogenated rosin triglyceride (hydrogenated ester gum), rosin triglyceride (ester gum), glyceryl behenate/eicosadioate, trimethylol propane trioctanoate, trimethylol propane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated pentaerythrityl rosinate, ditrimethylol propane triethyl hexaneoate, ditrimethylol propane (isostearate/sebacate), pentaerythrityl triethyl hexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, and glycol distearate (ethylene glycol distearate); derivatives of dimer acids or dimer diols such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dilinoleate, dimer dilinoleyl diisostearate, dimer dilinoleyl hydrogenated rosin condensate, dimer linoleic acid hard castor oil, and hydroxyalkyl dimer dilinoleyl ether; fatty acid alkanolamides such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamide (cocamide methyl MEA); silicones including amino-modified silicones such as dimethicone (dimethyl polysiloxane), highly polymerized dimethicone (highly polymerized dimethyl polysiloxane), cyclomethicone (cyclic dimethyl siloxane, decamethyl cyclopentasiloxane), phenyl trimethicone, diphenyl dimethicone, phenyl dimethicone, stearoxy propyl dimethyl amine, a (aminoethyl aminopropyl methicone/dimethicone) copolymer, dimethiconol, a dimethiconol cross polymer, a silicone resin, silicone rubber, aminopropyl dimethicone, and amodimethicone, polyether-modified silicones such as cation-modified silicone and dimethicone copolyol, polyglycerin-modified silicone, sugar-modified silicone, carboxylic acid-modified silicone, phosphoric acid-modified silicone, sulfuric acid-modified silicone, alkyl-modified silicone, fatty acid-modified silicone, alkyl ether-modified silicone, amino acid-modified silicone, peptide-modified silicone, fluorine-modified silicone, cation-modified and polyether-modified silicone, amino-modified and polyether-modified silicone, alkyl-modified and polyether-modified silicone, and a polysiloxane-oxyalkylene copolymer; and fluorine-based oils such as perfluorodecane, perfluorooctane, and perfluoro polyether can be used.

As the moisturizing agent and the touch improving agent, for example, one or more kinds selected from polyhydric alcohols such as glycerin, propylene glycol, 1,3-butylene glycol, sorbitol, polyglycerin, polyethylene glycol, and dipropylene glycol; water-soluble polymer such as NMF components such as sodium lactate, hyaluronic acid, collagen, mucopolysaccharides, and chondroitin sulfate; polyols such as glycerin, 1,3-butylene glycol, propylene glycol, 3-methyl-1,3-butanediol, 1,3-propanediol, 2-methyl-1,3-propanediol, trimethylol propane, pentaerythritol, hexylene glycol, diglycerin, polyglycerin, diethylene glycol, polyethylene glycol, dipropylene glycol, polypropylene glycol, and an ethylene glycol-propylene glycol copolymer, and polymers thereof; glycol alkyl ethers such as diethylene glycol monoethyl ether (ethoxydiglycol), ethylene glycol monoethyl ether, ethylene glycol monobutyl ether, and diethylene glycol dibutyl ether; sugar alcohols such as sorbitol, xylitol, erythritol, mannitol, and maltitol; saccharides such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β-, and γ-cyclodextrins, and cyclodextrins obtained by modification such as maltosylation, hydroxyalkylation, or the like), glucan, chitin, chitosan, heparin and derivatives thereof, pectin, arabinogalactan β, dextrin, dextran, glycogen, ethyl glucoside, and a glucosyl ethyl methacrylate polymer or a copolymer, and derivatives thereof; hyaluronic acid and sodium hyaluronate; sodium chondroitin sulfate; mucoitinsulfuric acid, charonin sulfate, keratosulfate, dermatan sulfate; snow fungus extract, and snow fungus polysaccharide; fucoidan; tuberosa polysaccharide, and naturally derived polysaccharide; organic acids such as citric acid, tartaric acid, and lactic acid, and salts thereof; urea; 2-pyrrolidone-5-carboxylic acid and salts thereof such as a sodium salt thereof; amino acids such as betaine (trimethyl glycine), proline, hydroxyproline, arginine, lysine, serine, glycine, alanine, phenylalanine, tyrosine, β-alanine, threonine, glutamic acid, glutamine, asparagine, aspartic acid, cysteine, cystine, methionine, leucine, isoleucine, valine, tryptophan, histidine, and taurine, and salts thereof; protein peptides such as collagen, fish-derived collagen, atelocollagen, gelatin, elastin, collagen decomposed peptide, hydrolyzed collagen, hydroxypropyl ammonium chloride hydrolyzed collagen, elastin decomposed peptide, keratin decomposed peptide, hydrolyzed keratin, conchiolin decomposed peptide, hydrolyzed conchiolin, silk proteolytic peptide, hydrolyzed silk, lauroyl hydrolyzed silk sodium, soybean proteolytic peptide, wheat proteolytic peptide, hydrolyzed wheat protein, casein decomposed protein, and acylated peptide, and derivatives thereof; acylated peptides such as palmitoyl oligopeptide, palmitoyl pentapeptide, and palmitoyl tetrapeptide; silylated peptides; animal-plant extract components such as a lactic acid bacteria culture solution, yeast extract, egg shell membrane protein, bovine submaxillary gland mucin, hypotaurine, sesame lignan glycoside, glutathione, albumin, whey, choline chloride, and phosphoryl choline; placenta extract, elastin, collagen, aloe extract, hamamelis water, loofah water, chamomilla extract, licorice extract, comfrey extract, silk extract, rosa roxburghii extract, yarrow extract, eucalyptus extract, and melilot extract, ceramides such as natural type ceramides (types 1, 2, 3, 4, 5, and 6), hydroxyceramide, pseudo-ceramide, sphingoglycolipid, and ceramide- and sugar ceramide-containing extract can be used.

Examples of the surfactant include anionic surfactants such as a lauryl sulfuric acid ester salt, polyoxyethylene alkyl ether sulfate, an alkyl benzene sulfonate, polyoxyethylene alkyl ether phosphoric acid, polyoxyethylene alkyl phenyl ether phosphoric acid, N-acylamino acid salt, sodium stearate, potassium palmitate, sodium cetyl sulfate, sodium lauryl sulfate, triethanolamine palmitate, sodium polyoxyethylene lauryl phosphate, sodium acyl glutamate, and surfactin; cationic surfactants such as benzalkonium chloride, benzethonium chloride, stearyl trimethylammonium chloride, distearyl dimethylammonium chloride, and stearyl dimethylbenzyl ammonium chloride; amphoteric surfactants such as alkyldiaminoethylglycine hydrochloride, 2-alkyl-N-carboxymethyl-N-hydroxyethyl imidazolinium betaine, lauryl dimethyl amino acetic acid betaine, and lecithin; nonionic surfactants such as polyol fatty acid ester, glyceryl monostearate, lipophilic glyceryl monooleate, ethylene glycol monostearate, propylene glycol monostearate, sorbitan fatty acid ester, polyoxyethylene sorbitan fatty acid ester, polyoxyethylene fatty acid ester, polyoxyethylene alkyl ether, polyoxyethylene alkyl phenol ether, polyoxyethylene sorbitol fatty acid ester, N-acylamino acid ester, sucrose fatty acid ester, fatty acid alkylolamide, polyoxyethylenized sterol, polyoxyethylenized lanolin, and polyoxyethylene hard castor oil; fatty acid salts such as potassium laurate and potassium myristate; alkyl sulfuric acid ester salts such as sodium lauryl sulfate, triethanolamine lauryl sulfate, and ammonium lauryl sulfate; polyoxyethylene alkyl sulfates such as sodium laureth sulfate and triethanolamine laureth sulfate; acyl N-methyl amino acid salts such as sodium cocoyl methyl taurine, potassium cocoyl methyl taurine, sodium lauroyl methyl taurine, sodium myristoyl methyl taurine, sodium lauroyl methyl alanine, sodium lauroyl sarcosine, triethanolamine lauroyl sarcosine, and methyl alanine sodium lauroyl glutamate; acylamino acid salts such as sodium cocoyl glutamate, triethanolamine cocoyl glutamate, sodium lauroyl glutamate, sodium myristoyl glutamate, sodium stearoyl glutamate, ditriethanolamine palmitoyl aspartate, and triethanolamine cocoyl alanine; polyoxyethylene alkyl ether acetates such as sodium laureth acetate; succinic acid ester salts such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates such as hard coconut oil fatty acid glycerin sodium sulfate; alkyl benzene polyoxyethylene sulfate; olefin sulfonates such as sodium α-olefin sulfonate; alkyl sulfosuccinates such as lauryl disodium sulfosuccinate, and sodium dioctyl sulfosuccinate; alkyl ether sulfosuccinates such as laureth disodium sulfosuccinate, monolauroyl monoethanolamide polyoxyethylene sulfosuccinic acid sodium, and lauryl polypropylene glycol sulfosuccinic acid sodium; alkyl benzene sulfonates such as sodium tetradecyl benzene sulfonate and triethanolamine tetradecyl benzene sulfonate; alkyl naphthalene sulfonates; alkane sulfonates; α-sulfo fatty acid methyl ester salt; acyl isethionates; alkyl glycidyl ether sulfonates; alkyl sulfoacetates; alkyl ether phosphoric acid ester salts such as sodium laureth phosphate, sodium dilaureth phosphate, sodium trilaureth phosphate, and sodium monooreth phosphate; alkyl phosphoric acid ester salts such as potassium lauryl phosphate; sodium caseinate; alkyl aryl ether phosphates; fatty acid amide ether phosphate; phospholipids such as phosphatidyl glycerol, phosphatidyl inositol, and phosphatidic acid; anionic surfactants including silicone-based anionic surfactants such as carboxylic acid-modified silicone, phosphoric acid-modified silicone, and sulfuric-acid modified silicone; polyoxyethylene alkyl ethers having various polyoxyethylene addition numbers such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers), and octyldodeceths (polyoxyethylene octyl dodecyl ethers); polyoxyethylene alkyl phenyl ethers; castor oil and hard castor oil derivatives such as polyoxyethylene hard castor oil, polyoxyethylene castor oil, polyoxyethylene hard castor oil monoisostearate, polyoxyethylene hard castor oil triisostearate, polyoxyethylene hard castor oil monopyroglutamic acid monoisostearic acid diester, and polyoxyethylene hard castor oil maleic acid; polyoxyethylene phytosterol; polyoxyethylene cholesterol; polyoxyethylene cholestanol; polyoxyethylene lanolin; polyoxyethylene reduced lanolin; polyoxyethylene-polyoxypropylene alkyl ethers such as polyoxyethylene-polyoxypropylene cetyl ether, polyoxyethylene-polyoxypropylene 2-decyl tetradecyl ether, polyoxyethylene-polyoxypropylene monobutyl ether, polyoxyethylene-polyoxypropylene hydrogenated lanolin, and polyoxyethylene-polyoxypropylene glycerin ether; polyoxyethylene-polyoxypropylene glycol; (poly)glycerin polyoxypropylene glycols such as PPG-9 diglyceryl; glycerin fatty acid partial esters such as glyceryl stearate, glyceryl isostearate, glyceryl palmitate, glyceryl myristate, glyceryl oleate, coconut oil fatty acid glyceryl ester, mono cottonseed oil fatty acid glycerin ester, glyceryl monoerucate, glyceryl sesquioleate, α,α'-oleic acid pyroglutamic acid glycerin ester, and monostearic acid glycerin maleic aicd ester; polyglycerol fatty acid esters such as polyglyceryl-2 stearate, polyglyceryl-3 stearate, polyglyceryl-4 stearate, polyglyceryl-5 stearate, polyglyceryl-6 stearate, polyglyceryl-8 stearate, and polyglyceryl-10 stearate, polyglyceryl-6 distearates, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate; ethylene glycol mono fatty acid esters such as ethylene glycol monostearate; propylene glycol mono fatty acid esters such as propylene glycol glycol monostearate; pentaerythritol partial fatty acid esters; sorbitol partial fatty acid esters; maltitol partial fatty acid esters; maltitol ethers; sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, pent-2-ethylhexyl acid diglycerol sorbitan, and tetra-2-ethylhexyl acid diglycerol sorbitan; and sugar derivative partial esters such as sucrose fatty acid ester, methyl glucoside fatty acid ester, and trehalose undecylenate.

Other examples of the surfactant include alkyl glucosides such as caprylyl glucoside; alkyl polyglycosides; lanolin alcohols; reduced lanolins; polyoxyethylene fatty acid mono- and diesters such as polyoxyethylene distearate, polyethylene glycol diisostearate, polyoxyethylene monooleate, and polyoxyethylene dioleate; polyoxyethylene-propylene glycol fatty acid esters; polyoxyethylene glycerin fatty acid esters including polyoxyethylene monooleates such as polyoxyethylene glycerin monostearate, polyoxyethylene glycerin monoisostearate, and polyoxyethylene glycerin triisostearate; polyoxyethylene sorbitan fatty acid esters such as polyoxyethylene sorbitan monooleate, polyoxyethylene sorbitan monostearate, polyoxyethylene sorbitan monooleate, and polyoxyethylene sorbitan tetraoleate; polyoxyethylene sorbitol fatty acid esters such as polyoxyethylene sorbitol monolaurate, polyoxyethylene sorbitol monooleate, polyoxyethylene sorbitol pentaoleate, and polyoxyethylene sorbitol monostearate; polyoxyethylene methyl glucoside fatty acid esters; polyoxyethylene alkyl ether fatty acid esters; polyoxyethylene animal and plant fats and oils such as polyoxyethylene sorbitol beeswax; alkyl glyceryl ethers such as isostearyl glyceryl ether, chimyl alcohol, selachyl alcohol, and batyl alcohol; polyhydric alcohol alkyl ethers; polyoxyethylene alkyl amines; tetrapolyoxyethylene-tetrapolyoxypropylene-ethylenediamine condensates; natural-based surfactants such as saponin and sophorolipid; polyoxyethylene fatty acid amides; fatty acid alkanolamides such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methyl ethanolamide (cocamide methyl MEA); alkyl dimethyl amine oxides such as lauramine oxide, cocamine oxide, stearamine oxide, and behenamine oxide; alkyl ethoxy dimethyl amine oxides; polyoxyethylene alkyl mercaptan; nonionic surfactants including silicone-based nonionic surfactants such as polyether-modified silicones such as dimethicone copolyol, a polysiloxane-oxyalkylene copolymer, polyglycerin-modified silicone, and sugar-modified silicone; cationic surfactants such as alkyl trimethyl ammonium chlorides such as behentrimonium chloride, steartrimonium chloride, setrimonium chloride, and lauryltrimonium chloride; alkyl trimethylammonium bromides such as stearyltrimonium bromide; dialkyl dimethyl ammonium chlorides such as distearyldimonium chloride and dicocodimonium chloride; fatty acid amidoamines such as stearamidopropyl dimethylamine and stearamidoethyl diethylamine and salts thereof; alkyl ether amine such as stearoxypropyl dimethyl amine and salts or quaternary salts thereof; fatty acid amide type quaternary ammonium salts such as ethyl sulfuric acid long chain branched fatty acid (12 to 31) amino propyl ethyl dimethyl ammonium and ethyl sulfuric acid lanolin fatty acid amino propyl ethyl dimethyl ammonium; polyoxyethylene alkyl amines and salts or quaternary salts thereof; alkyl amine salts; fatty acid amide guanidinium salts; alkyl ether ammonium salts; alkyl trialkylene glycol ammonium salts; benzalkonium salts; benzethonium salts; pyridinium salts such as cetylpyridinium chloride; imidazolinium salts; alkyl isoquinolinium salts; dialkyl moriphonium salts; polyamine fatty acid derivatives; amino-modified silicones such as aminopropyl dimethicone and amodimethicone, silicone-based cationic surfactants such as cation-modified silicone, cation-modified and polyether-modified silicones, and amino-modified and polyether-modified silicones; N-alkyl-Ns such as lauryl betaine (lauryl dimethyl amino acetic acid betaine), and N-dimethyl amino acid betaine; fatty acid amidoalkyl-Ns such as cocamidopropyl betaine and lauramidopropyl betaine, and N-dimethyl amino acid betaine; imidazoline type betaines such as sodium cocoamphoacetate and sodium lauroamphoacetate; alkyl sulfobetaines such as alkyl dimethyl taurine; sulfuric acid type betaines such as alkyl dimethyl amino ethanol sulfuric acid ester; phosphoric acid type betaines such as alkyl dimethyl amino ethanol phosphoric acid ester; sphingophospholipids such as phosphatidyl choline, phosphatidyl ethanolamine, phosphatidyl serine, and sphingomyelin, phospholipids such as lysolecithin, hydrogenated soybean phospholipid, partially hydrogenated soybean phospholipid, hydrogenated egg yolk phospholipid, partially hydrogenated egg yolk phospholipid, and hydroxide lecithin; amphoteric surfactants such as silicone-based amphoteric surfactants; polyvinyl alcohol, sodium alginate, starch derivatives, tragacanth gum, and an acrylic acid-methacrylic acid alkyl copolymer; and polymer surfactants such as various silicone-based surfactants.

As the surfactant, one or more kinds selected from the above-described compounds can be used.

As the polymerizing-thickening-gelling agent, for example, one or more kinds selected from guar gum, locust bean gum, queens seed, carrageenan, galactan, gum arabic, tara gum, tamarind, furcellaran, karaya gum, hibiscus, chara gum, tragacanth, pectin, pectic acid and salts thereof such as a sodium salt thereof, alginic acid and salts thereof such as a sodium salt thereof, and mannan; starches of rice, corn, potato, and wheat; xanthan gum, dextran, succinoglucan, curdlan, hyaluronic acid and salts thereof, xanthan gum, pullulan, gellan gum, chitin, chitosan, agar, brown alga extract, chondroitin sulfate, casein, collagen, gelatin, and albumin; methyl cellulose, ethyl cellulose, hydroxyethyl cellulose, hydroxypropyl cellulose, carboxymethyl cellulose and salts thereof such as a sodium salt thereof, and celluloses such as methyl hydroxypropyl cellulose, sodium cellulose sulfate, dialkyl dimethyl ammonium cellulose sulfate, crystalline cellulose, and cellulose powder, and derivatives thereof; starch-based polymers such as soluble starch, carboxymethyl starch, methyl hydroxypropyl starch, and methyl starch, and starch derivatives such as starch hydroxypropyltrimonium chloride and aluminum corn starch octenylsuccinate; alginic acid derivatives such as sodium alginate and alginic acid propylene glycol ester; polyvinylpyrrolidone (PVP), polyvinyl alcohol (PVA), a vinylpyrrolidone-vinyl alcohol copolymer, and polyvinyl methyl ether; polyethylene glycol, polypropylene glycol, and a polyoxyethylene-polyoxypropylene copolymer; amphoteric methacrylic acid ester copolymers such as a (methacryloyloxyethyl carboxybetaine/alkyl methacrylate) copolymer and an (acrylate/stearyl acrylate/ethylamine oxide methacrylate) copolymer; a (dimethicone/vinyl dimethicone) cross polymer, an (alkyl acrylate/diacetone acrylamide) copolymer, and an (alkyl acrylate/diacetone acrylamide) copolymer AMP; a polyvinyl acetate partial saponification product and a maleic acid copolymer; a vinylpyrrolidone-dialkyl aminoalkyl methacrylate copolymer; acrylic resin alkanolamine; polyester and water-dispersible polyester; polyacrylamide; plyacrylic acid ester copolymers such as ethyl polyacrylate, a carboxyvinyl polymer, polyacrylic acid and salts thereof such as a sodium salt thereof, and an acrylic acid-methacrylic acid ester copolymer; acrylic acid-alkyl methacrylate copolymers; cationized celluloses such as polyquaternium-10, diallyl dimethyl ammonium chloride-acrylamide copolymers such as polyquaternium-7, acrylic acid-diallyl dimethyl ammonium chloride copolymers such as polyquaternium-22, acrylic acid-diallyl dimethyl ammonium chloride-acryl amide copolymer such as polyquaternium-39, an acrylic acid-cationized methacrylic acid ester copolymer, an acrylic acid-cationized methacrylic acid amide copolymer, an acrylic acid-methyl acrylate-methacrylamide propyl trimethyl ammonium copolymer such as polyquaternium-47, and a methacrylate chloride choline ester copolymer; cationized polysaccharides such as cationized oligosaccharide, cationized dextran, and guar hydroxypropyl trimonium chloride; polyethyleneimine; cationic polymers; copolymers with a polymer of 2-methacryloyloxyethyl phosphorylcholine such as polyquaternium-51 or an butyl methacrylate copolymer; polymer emulsions such as an acrylic resin emulsion, polyacrylic acid ethyl emulsion, an polyacrylic alkyl ester emulsion, a polyvinyl acetate resin emulsion, natural rubber latex, and synthetic latex; nitrocellulose; polyurethanes and various copolymers; various silicones; various silicone-based copolymers such as an acryl-silicone graft copolymer; various fluorine-based polymers; 12-hydroxystearic acid and salts thereof; dextrin fatty acid esters such as dextrin palmitic acid ester, dextrin stearic acid ester, and dextrin 2-ethyl hexanoic acid palmitic acid ester; dextrin fatty acid esters such as dextrin myristate; organically modified clay minerals such as silicic anhydride, fumed silica (ultrafine particle silicic anhydride), aluminum magnesium silicate, sodium magnesium silicate, a metal soap, dialkyl phosphoric acid metal salt, bentonite, hectorite, dimethyl benzyldodecyl ammonium montmorillonite clay, and dimethyl dioctadecyl ammonium montmorillonite clay, sucrose fatty acid esters such as sucrose palmitic acid ester and sucrose stearic acid ester, amino acid derivatives such as N-lauroyl-L-glutamic acid and α, γ-di-n- butylamine, benzylidene derivatives of sorbitol such as monobenzylidene sorbitol and dibenzylidene sorbitol, and fructooligosaccharide fatty acid ester can be used.

As the solvent and the propellant, for example, one or more kinds selected from monohydric alcohols such as methanol, ethanol, propanol, benzyl alcohol, phenethyl alcohol, isopropyl alcohol, isobutyl alcohol, hexyl alcohol, 2-ethyl hexanol, cyclohexanol, octyl alcohol, butanol, and pentanol; ketones such as acetone and methyl ethyl ketone; lower alcohols such as ethanol, 2-propanol (isopropyl alcohol), butanol, and isobutyl alcohol; glycols such as propylene glycol, 1,3-butylene glycol, diethylene glycol, dipropylene glycol, and isopentyldiol; glycol ethers such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, and dipropylene glycol monoethyl ether; glycol ether esters such as ethylene glycol monoethyl ether acetate, diethylene glycol monoethyl ether acetate, and propylene glycol monomethyl ether acetate; glycol esters such as diethoxyethyl succinate and ethylene glycol disuccinate; benzyl alcohol, benzyloxyethanol, propylene carbonate, dialkyl carbonate, acetone, ethyl acetate, and N-methyl pyrrolidone; toluene; fluorocarbon and next generation freon; LPG, dimethyl ether, carbon dioxide, cellosolve, dioxane, dimethyl formamide, dimethyl sulfoxide, and crotamiton can be used.

As the antioxidant, the reductant, and the oxidant, for example, one or more kinds selected from tocopherol (vitamin E) and tocopherol derivatives such as tocopherol acetate; BHT and BHA; gallic acid derivatives such as propyl gallate; vitamin C (ascorbic acid) and/or derivatives thereof; erythorbic acid and derivatives thereof; sulfites such as sodium sulfite; bisulfite such as sodium bisulfite; thiosulfates such as sodium thiosulfate; meta hydrogensulfite; thiotaurine and hypotaurine; thioglycerol, thiourea, thioglycolic acid, cysteine hydrochloride, thioglycolic acid, cysteine, and cysteamine; hydrogen peroxide water, ammonium persulfate, sodium bromate, percarbonate, nordihydroguaiaretic acid, a guaiac resin, butyl hydroxyanisole, dibutyl hydroxytoluene (BHT), and 2,2'-methylenebis(4-methyl-6-t-butyl)phenol can be used.

As the preservative, the antibacterial agent, and the chelating agent, for example, one or more kinds selected from hydroxybenzoic acids such as methylparaben, ethylparaben, propylparaben, and butylparaben and salts thereof or esters thereof, salicylic acid, sodium benzoate, phenoxyethanol, 1,2-diols such as 1,2-pentanediol and 1,2-hexanediol, isothiazolinone derivatives such as methyl chloroisothiazolinone and methyl isothiazolinone, imidazolinium urea, dehydroacetic acid and salts thereof, phenols, halogenated bisphenols such as triclosan, acid amide, a quaternary ammonium salt, trichlorocarbanilide, zinc pyrithione, benzalkonium chloride, benzethonium chloride, sorbic acid, chlorhexidine, chlorhexidine gluconate, halocarban, hexachlorophene, hinokitiol, phenol, other phenols such as isopropyl phenol, cresol, thymol, parachlorophenol, phenylphenol, sodium phenylphenol, phenyl ethyl alcohol, photosensitizers, antibacterial zeolite, silver ion, edetates (ethylene diamine tetra-acetic acid salt) such as EDTA, EDTA2Na, EDTA3Na, and EDTA4Na, hydroxyethyl ethylenediamine triacetates such as HEDTA3Na, pentetate (diethylenetriamine pentaacetate); phytic acid; phosphonic acids such as etidronic acid, and salts thereof such as a sodium salt thereof, sodium oxalate, polyamino acids such as polyaspartic acid and polyglutamic acid, sodium polyphosphate, sodium metaphosphate, and phosphoric acid; sodium citrate, citric acid, alanine, dihydroxyethylglycine, gluconate, ascorbic acid, succinic acid, tartaric acid, pyrophosphate, and hexametaphosphate can be used.

As the pH adjusting agent, the acid, and alkali, for example, one or more kinds selected from alkali metal hydroxides and alkaline earth metal hydroxides such as sodium hydroxide, potassium hydroxide, calcium hydroxide, magnesium hydroxide, 2-amino-2-methyl-1,3-propanediol, 2-amino-2-hydroxymethyl-1,3-propanediol, arginine, guanidine carbonate, ammonium carbonate, ammonia, ammonia water, triethanolamine, dimethyl amine, diethylamine, trimethylamine, triethylamine, triisopropanolamine, trisodium phosphate, disodium hydrogen phosphate, dipotassium hydrogen phosphate, monoethanolamine, diethanolamine, isopropanol amine, diisopropanolamine, and polyethanolamine, alkalis such as primary, secondary, or tertiary alkyl amines, or primary, secondary, or tertiary alkanolamine, acids such as citric acid, tartaric acid, lactic acid, glycolic acid, hydrochloric acid, nitric acid, citric acid, sodium citrate, sodium lactate, succinic acid, acetic acid, sodium acetate, fumaric acid, sulfuric acid, malic acid, and phosphoric acid, polymers exhibiting acidity or alkalinity, such as alginic acid, polyglutamic acid, polyaspartic acid, a starch-acrylic acid graft polymer, polyacrylic acid, a polyvinyl acetate-crotonic acid copolymer, a vinyl acetate-(meth) acrylic acid copolymer, a vinyl acetate-crotonic acid copolymer, polyvinyl sulfonic acid, polyitaconic acid, a styrene-maleic anhydride copolymer, and acrylamide-acrylic acid copolymer can be used.

As the powder and the inorganic salt, for example, one or more kinds selected from inorganic powders having various sizes and shapes, such as mica, talc, kaolin, sericite, montmorillonite, kaolinite, mica, white mica, phlogopite, synthetic mica, lepidolite, biotite, vermiculite, magnesium carbonate, calcium carbonate, aluminum silicate, barium silicate, calcium silicate, magnesium silicate, strontium silicate, metal tungstate, magnesium, zeolite, barium sulfate, calcined calcium sulfate, calcium phosphate, fluorine apatite, hydroxyapatite, a ceramic powder, bentonite, smectite, clay, mud, a metal soap (for example, zinc myristate, calcium palmitate, and aluminum stearate), calcium carbonate, red oxide, yellow iron oxide, black iron oxide, ultramarine blue, prussian blue, carbon black, titanium oxide, fine or ultrafine particles of titanium oxide, zinc oxide, fine and ultrafine particles of zinc oxide, alumina, silica, fumed silica (ultrafine particle silicic anhydride), mica titanium, argentine, boron nitride, a photochromic pigment, synthetic fluorine phlogopite, a fine particle composite powder, gold, and aluminum, and inorganic powders such as powders hydrophobized or hydrophilizied by treating these with various surface treatment agents such as silicones such as hydrogen silicone and cyclic hydrogen silicone, or other silanes or a titanate coupling agent; starch, cellulose, a nylon powder, a polyethylene powder, polymethyl methacrylate powder, polystyrene powder, a copolymer resin powder of styrene and acrylic acid, a polyester powder, a benzoguanamine resin powder, a polyethylene terephthalate-polymethyl methacrylate laminated powder, a polyethylene terephthalate-aluminum-epoxy laminated powder, a urethane powder, a silicone powder, organic powders and surface treatment powders having various sizes and shapes such as a teflon (registered trademark) powder, and an organic-inorganic composite powder; sodium chloride-containing salts such as salt, crude salt, rock salt, sea salt, and natural salt; potassium chloride, aluminum chloride, calcium chloride, magnesium chloride, bittern, zinc chloride, and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate, and copper sulfate; sodium phosphates such as monosodium, disodium, trisodium phosphates, potassium phosphates, calcium phosphates, magnesium phosphate, magnesium oxide, lithia mica, silicic acid, aluminum magnesium silicate, vermiculite, higilite, a ceramic powder, dibasic calcium phosphate, aluminum hydroxide, and boron nitride; organic powders such as a polyamide powder, a polyester powder, a polyethylene powder, a polypropylene powder, a polystyrene powder, a polyurethane, a benzoguanamine powder, a polymethyl benzoguanamine powder, a tetrafluoroethylene powder, a polymethyl methacrylate powder, a silk powder, a nylon powder, 12 nylon, 6 nylon, a styrene-acrylic acid copolymer, a divinyl benzene-styrene copolymer, a vinyl resin, a urea resin, a phenol resin, a fluorine resin, a silicon resin, an acrylic resin, a melamine resin, an epoxy resin, a polycarbonate resin, a microcrystalline fiber powder, and lauroyl lysine; colored pigments including inorganic red pigments such as iron oxide, iron hydroxide, and titanium iron, inorganic brown pigments such as γ-iron oxide, inorganic yellow pigments such as yellow iron oxide and ocher, inorganic black pigments such as black iron oxide and carbon black, inorganic purple pigments such as manganese violet and cobalt violet, inorganic green pigments such as chromium hydroxide, chromium oxide, cobaly oxide, and cobalt titanate, inorganic blue pigments such as prussian blue and ultramarine blue, a powder obtained by laking a tar-based coloring matter, a powder obtained by laking a natural pigment, and a composite powder obtained by compositing these powders; pearl pigments such as titanium oxide-coated mica, titanium oxide-coated mica, bismuth oxychloride, titanium oxide-coated bismuth oxychloride, titanium oxide-coated talc, argentine, and titanium oxide-coated colored mica; metal powder pigments such as an aluminum powder, a copper powder, and a stainless steel powder; tar coloring matters such as Red Nos. 3, 104, 106, 201, 202, 204, 205, 220, 226, 227, 228, 230, 401, and 505, Yellow Nos. 4, 5, 202, 203, 204, and 401, Blue Nos. 1, 2, 201, and 404, Green Nos. 3, 201, 204, and 205, and Orange Nos. 201, 203, 204, 206, and 207; a powder selected from natural coloring matters such as carmine acid, laccaic acid, carthamin, brazilin, and crocin, and powders obtained by performing a surface treatment on these powders with a composite compound, oil, silicone, or a fluorine compound, montmorillonite, silicic anhydride, gypsum, diatomaceous earth, calcium carbonate, hydrotalcite, talc, glass, kaolin, a metal soap, aerosil, bismuth oxychloride, argentine, zinc white, and titanium dioxide can be used.

As the ultraviolet absorbent, for example, one or more kinds selected from benzoic acid-based ultraviolet absorbents such as para-aminobenzoic acid, para-aminobenzoic acid mono glycerin ester, N,N-dipropoxy para-aminobenzoic acid ethyl ester, N,N-diethoxy para-aminobenzoic acid ethyl ester, N,N-dimethyl para-aminobenzoic acid ethyl ester, N,N-dimethyl-para-aminobenzoic acid butyl ester, and N,N-dimethyl para-aminobenzoic acid ethyl ester; anthranilic acid-based ultraviolet absorbants such as homomenthyl-N-acetyl anthranilate; salicylic acid-based ultraviolet absorbents such as salicylic acid and sodium salts thereof, amyl salicylate, menthyl salicylate, homomenthyl salicylate, octyl salicylate, phenyl salicylate, and benzyl salicylate, p-isopropanol phenyl salicylate; cinnamic acid-based ultraviolet absorbents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, 2-ethylhexyl p-methoxycinnamate (octyl para-methoxycinnamate), 2-ethoxyethyl-p-methoxycinnamate (cinoxate), cyclohexyl-p-methoxycinnamate, ethyl-α-cyano-β-phenyl cinnamate, 2-ethylhexyl α-cyano-β-phenyl cinnamate (octocrylene), glyceryl mono-2-ethylhexanoyl-di-para-methoxycinnamate, and ferulic acid, and derivatives thereof; benzophenone-based ultraviolet absorbents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methyl benzophenone, 2-hydroxy-4-methoxybenzophenone-5-sulfonate,4-phenyl benzophenone, 2-ethylhexyl-4'-phenyl-benzophenone-2-carboxylate, 2-hydroxy -4-n-octoxybenzophenone, and 4-hydroxy-3-carboxybenzophenone; 3-(4'-methylbenzylidene)-d,1-camphor, 3-benzylidene-d,1-camphor; 2-phenyl-5-methyl benzoxazole; 2,2'-hydroxy-5-methyl phenyl benzotriazole; 2-(2'-hydroxy-5'-t-octylphenyl)benzotriazole; 2-(2'-hydroxy-5'-methylphenyl benzotriazole; dibenzaladine; dianisoyl methane; 5-(3,3-dimethyl-2-norbornylidene)-3-pentan-2-one; and dibenzoylmethane derivatives such as 4-t-butyl methoxydibenzoylmethane; octyl triazone; urocanic acid derivatives such as urocanic acid and ethyl urocanate; 2-(2'-hydroxy-5'-methylphenyl)benzotriazole, 1-(3,4-dimethoxyphenyl)-4,4-dimethyl-1,3-pentanedione, hydantoin derivatives such as 2-ethylhexyl dimethoxybenzylidene dioxoimidazolidine propionate, phenylbenzimidazole sulfonic acid, terephthalyl dicamphor sulfonic acid, drometrizole trisiloxane, methyl anthranilate, rutin and derivatives thereof, and oriyzanol and derivatives thereof can be used.

As the whitening agent, for example, one or more kinds selected from hydroquinone glycosides such as arbutin and α-arbutin, and esters thereof; ascorbic acid phosphoric acid ester salts such as ascorbic acid, an ascorbic acid phosphoric acid ester sodium salt, and an ascorbic acid phosphoric acid ester magnesium salt, ascorbic acid fatty acid ester such as ascorbic acid tetra isopalmitic acid ester, ascorbic acid alkyl ether such as ascorbic acid ethyl ether, ascorbic acid glucoside such as ascorbic acid 2-glucoside and fatty acid esters thereof, ascorbic acid derivatives such as ascorbic acid sulfuric acid ester and tocopheryl ascorbyl phosphate; kojic acid, ellagic acid, tranexamic acid and derivatives thereof, ferulic acid and derivatives thereof, placenta extract, glutathione, orizanol, butyl resorcinol, oil-soluble chamomilla extract, oil-soluble licorice extract, Chinese tamarisk extract, saxifrage extract, glabridin, glabrene, liquiritin, isoliquiritin, and licorice extract containing these, placenta extract, carotenoid and animal and plant extracts containing these, neoagarobiose, agarose oligosaccharide, asparagus extract, bistort extract, pea extract, rose fruit extract, scutellaria root extract, ononis spinosa extract, seaweed extract, raspberry extract, sophora root extract, mucuna birdwoodiana extract, gokahi extract, vegetable oil containing linoleic acid, saisin extract, hawthorn extract, cassia nomame extract, white lily extract, peony extract, inula flower extract, mulberry bark extract, soybean extract, tea extract, angelica extract, molasses extract, white lotus extract, beech extract, grape seed extract, flor de manita extract, hop extract, rosa rugosa extract, mokka extract, saxifrage extract, coix seed extract, and Momordica grosvenori extract, and extracts of plants such as asparagus, madder, red grape, mallotus, akebi, cannabis, morning glory, adzuki bean, gambir, sweet hydrangea, pentaphyllum, Japanese knotweed, fig tree, ginkgo, ylang-ylang, prunella, apricot, bearberry, satsuma mandarin, siberian ginseng, sicklepod, sophora, peas, plantain, okra, ragweed, juglans, golden lace, strawberry, kaki, ground ivy, polygonum, cashew, valerian, snake gourd, karin, guarana, bellflower, chrysanthemum, catalpa, sorrel, gymnema sylvestre, agrimonia, guava, wolfberry, arrowroot, camphor tree, chestnut, mucuna birdwoodiana, bay, cinnamon, raspberry, pepper, coffee, figwort, colombo, sasanqua, Japanese pepper, saffron, cherry tree, pomegranate, sophorae subprostrata radix, chamaecrista nomame, aster, calamus, watermelon, stevia, plum, english ivy, european pear, yarrow, juniperus communis, horseradish, sweet flag, water dropwort, senega, senna, rhubarb, bitter orange, tamarind, aralia elata, dandelion, chicory, clove, schisandra chinensis, umbrella polypore sclerotium, evening primrose, centella asiatica, dayflower, tetragonia tetragonoides, juglans regia, wax gourd, hardy rubber tree, sunset hibiscus, shepherd's purse, summer orange, nandina domestica thumb, picrasma wood, siberian yarrow, pineapple, hibiscus, papaya, basil, lotus, barley, blackberry lily, peanut, isodon japonicus, water caltrops, pistachio, cypress, agaricus blazei murill, angelicae dahurica, loquat, coltsfoot, Japanese sumac, thoroughwort, blueberry, saposhnikovia, ground cherry, magnolia, flowering quince, rosa maikwai, ephedra, mango, ganoderma lucidum, bupleurum, lythrum anceps, Japanese hornwort, mimosa, melilot, melon, lily magnolia, momordica-grosvenorii, Jews mallow, bean sprouts, bitter cardamon, leonurus, cornflower, palm, ulmus pumila, mistletoe, water pepper, pokeweed, bayberry, daphniphyllum macropodum, wormwood, rye, orchid, longan, apple, litchi, and weeoing forsythia can be used.

As the vitamins and derivatives thereof, for example, one or more kinds selected from vitamin A's such as retinol, retinol acetate, retinol palmitate; vitamin B's such as thiamine hydrochloride, thiamine sulfate, riboflavin, riboflavin acetate, riboflavin butyric acid ester, pyridoxine hydrochloride, pyridoxine dioctanoate, pyridoxine palmitate, flavin adenine dinucleotide, cyanocobalamin, folic acids, nicotinic acids such as nicotinic acid amide-benzyl nicotinate, and colines; vitamin C's such as ascorbic acid and salts of sodium and the like thereof; vitamin D; vitamin E's such as α, β, γ, and δ-tocopherols; other vitamins such as pantothenic acid and biotin; ascorbic acid phosphoric acid ester salts such as an ascorbic acid phosphoric acid ester sodium salt and an ascorbic acid phosphoric acid ester magnesium salt, ascorbic acid fatty acid ester such as ascorbic acid tetraisopalmitic acid ester-stearic acid ascorbyl-palmitic acid ascorbyl-dipalmitic acid ascorbyl, ascorbic acid alkyl ethers such as ascorbic acid ethyl ether, ascorbic acid glucosides such as ascorbic acid 2-glucoside, fatty acid esters thereof, and ascorbic acid derivatives such as phosphoric acid tocopheryl ascorbyl; vitamin derivatives including tocopherol derivatives such as tocopherol nicotinate, tocopherol acetate, tocopherol linoleate, tocopherol ferulate, and tocopherol phosphoric acid ester, and tocotrienol can be used.

As the antiphlogistic agent, the anti-inflammatory agent, the drug for hair growth, the blood circulation accelerator, the stimulant, the hormone, the anti-wrinkle agent, the anti-aging agent, the tightening agent, the cold sense agent, the warm sense agent, the wound healing accelerator, the irritation alleviating agent, the analgesic, and the cell activator, for example, one or more kinds selected from glycyrrhizic acid and derivatives thereof, glycyrrhetinic acid derivatives, salicylic acid derivatives, hinokitiol, guaiazulene, allantoin, indomethacin, zinc oxide, hydrocortisone acetate, prednisone, diphedoramine hydrochloride, and chlorpheniramine maleate; plant extracts such as peach leaf extract and mugwort extract; plant extracts or tinctures such as Japanese green gentian extract, pepper tincture, ginger tincture, ginger extract, and cantharis tincture; capsaicin, nonanoic acid vanillylamide, zingerone, ichthammol, tannic acid, borneol, cyclandelate, cinnarizine, tolazoline, acetylcholine, verapamil, cepharanthine, γ-orizanol, cepharanthine, vitamin E, and derivatives of tocopherol nicotinate·tocopherol acetate, γ-orizanol, derivatives of nicotinic acid, nicotinic acid amide·nicotinic acid benzyl ester·inositol hexanicotinate, nicotine alcohol, allantoin, photosensitizers 301 and 401, carpronium chloride, pentadecanoic acid monoglyceride, flavanonol derivatives, stigmasterol or stigmastanol and glycosides thereof, and minoxidil; estradiol, estrone, ethinyl estradiol, cortisone, hydrocortisone, and prednisone; retinols, retinoic acids, and tocopheryl retinoic acid; derivatives such as lactic acid, glycolic acid, gluconic acid, fruit acid, salicylic acid, and glycosides thereof esterification product, α- or β-hydroxy acids such as hydroxy capric acid, long-chain α-hydroxy fatty acid, and long-chain α-hydroxy fatty acid cholesteryl, and derivatives thereof; γ-amino butyric acid and γ-amino-β-hydroxybutyric acid; carnitine; creatine; ceramides and sphingosines; caffeine, xanthine, and derivatives thereof; antioxidants and active oxygen scavengers such as coenzyme Q10, carotene, lycopene, and astaxanthin; catechins; flavones such as quercetin; isoflavones; gallic acid and ester sugar derivatives; polyphenols such as tannin, sesamin, protoanthocyanidin, chlorogenic acid, and apple polyphenol; derivatives such as rutin and glycosides; derivatives such as hesperidin and glycosides; lignan glycoside; licorice extract-related substances such as glabridin, glabrene, liquiritin, and isoliquiritin; lactoferrin; shogaol and gingerol; fragrance substances and derivatives thereof such as menthol, camphor, and cedrol; capsaicin, vanillin, and derivatives thereof; insect repellents such as diethyltoluamide; and a complex of a physiologically active substance and cyclodextrin can be used.

As the plant, animal, and microbial extract, for example, one or more kinds selected from iris extract, ashitaba extract, thujopsis dolobrata extract, asparagus extract, avocado extract, sweet hydrangea leaf extract, almond extract, altea extract, arnica extract, aloe extract, apricot extract, apricot kernel extract, ginkgo extract, inchikow extract, fennel extract, turmeric extract, oolong tea extract, uva-ursi extract, rose fruit extract, echinacea leaf extract, isodon japonicus extract, scutellaria root extract, phellodendron bark extract, coptis japonica extract, barley extract, panax ginseng extract, hypericum extract, lamium album extract, ononis extract, Netherland mustard extract, orange extract, a dried seawater product, seaweed extract, persimmon leaf extract, pyracantha fortuneana fruit extract, hydrolyzed elastin, a hydrolyzed wheat powder, hydrolyzed silk, pueraria root extract, chamomilla extract, oil-soluble chamomilla extract, carrot extract, capillary artemisia extract, wild oat extract, hibiscus sabdariffa extract, licorice extract, oil-soluble licorice extract, kiwi extract, kiou apple extract, Jew's ear mushroom extract, cinchona bark extract, cucumber extract, paulownia tomentosa leaf extract, guanosine, guava extract, sophora root extract, gardenia extract oil, kuma bamboo grass extract, sophora flavescens extract, walnut extract oil, chestnut extract, grapefruit extract, clematis extract, black rice extract, brown sugar extract, black vinegar extract, chlorella extract, mulberry extract, gentian extract, geranium thunbergii extract, black tea extract, yeast extract, magnolia bark extract, coffee extract, burdock extract, rice extract, fermented rice extract, fermented rice bran extract, rice germ, comfrey extract, collagen, cowb extract, saisin extract, bupleurum root extract, umbilical cord extract liquid, saffron extract, salvia extract, saponaria officinalis extract, sasa extract, hawthorn extract, sansha extract, Japanese pepper extract, shiitake mushroom extract, rehmannia root extract, lithospermum root extract, perilla extract, Japanese linden extract, meadowsweet extract, jatoba extract, peony extract, ginger extract, calamus root extract, white birch extract, white Jew's ear mushroom extract, horsetail extract, stevia extract, a stevia-fermented product, Chinese tamarisk extract, English ivy extract, whitethorn extract, Sambucus nigra extract, yarrow extract, peppermint extract, sage extract, mallow extract, cnidium rhizome extract, Japanese green gentian extract, mulberry bark extract, rhubarb extract, soybean extract, jujube extract, thyme extract, dandelion extract, lichens extract, tea extract, clove extract, imperata extract, citrus unshiu peel extract, tea tree extract, Chinese blackberry extract, capsicum extract, Japanese angelica root extract, calendula officinalis extract, peach kernel extract, spruce extract, houttuynia extract, tomato extract, natto extract, carrot extract, garlic extract, eglantine extract, hibiscus extract, ophiopogon extract, lotus extract, parsley extract, birch extract, honey, hamamelis extract, parietaria extract, isodonjaponicus extract, bisabolol, cypress extract, lactobacillus bifidus extract, loquat extract, coltsfoot extract, petasites japonicus extract, hoelen extract, butcher bloom extract, grape extract, grape seed extract, propolis, loofah extract, safflower extract, peppermint extract, linden extract, tree peony extract, hop extract, rosa rugosa extract, pine extract, horse chestnut extract, skunk cabbage extract, soapberry extract, melissa extract, mozuku extract, peach extract, cornflower extract, eucalyptus extract, saxifrage extract, yuzu extract, lily extract, coix seed extract, wormwood extract, lavender extract, green tea extract, egg shell membrane extract, apple extract, rooibos tea extract, litchi extract, lettuce extract, lemon extract, weeping forsythia extract, astragalus sinicus extract, rose extract, rosemary extract, roman chamomile extract, royal jelly extract, and burnet extract can be used.

As the antipruritic agent, the keratin releasing-dissolving agent, the antiperspirant, the cooling agent, the astringent agent, the enzyme, and the nucleic acid, for example, one or more kinds selected from diphenhydramine hydrochloride, chlorpheniramine maleate, camphor, and a substance-P inhibitor; salicylic acid, sulfur, resorcinol, selenium sulfide, and pyridoxine; chlorohydroxyaluminum, aluminum chloride, zinc oxide, and zinc para-phenol sulfonate; menthol and methyl salicylate; citric acid, tartaric acid, lactic acid, aluminum potassium sulfate, and tannic acid; superoxide dismutase, catalase, lysozyme chloride, lipase, papain, pancreatin, and protease; ribonucleic acid and salts thereof, deoxyribonucleic acid and salts thereof, and adenosine triphosphate disodium can be used.

As the fragrance, for example, one or more kinds selected from vegetable perfumes such as mustard oil, orange oil, pepper oil, jasmine oil, cedar oil, calamus oil, terpin oil, neroli oil, rose oil, eucalyptus oil, lime oil, lemon oil, Japanese mint oil, and rosemary oil; animal perfumes such as musk, civet, castoreum, and ambergris; hydrocarbon-based perfumes such as bromostyrene, pinene, and limonene; alcohol-based perfumes such as benzyl alcohol and 1-menthol; ester-based perfumes such as ethyl acetate and methyl salicylate; aldehyde-based perfumes such as benzaldehyde and salicylaldehyde; ketone-based perfumes such as camphor, muscone, musk ketone, and 1-menthone; ether-based perfumes such as safrole; phenol-based perfumes such as thymol; lactone-based perfumes; acid-based perfumes such as phenylacetic acid; nitrogen compound-based perfumes such as indole; and synthetic perfumes, natural perfumes, and various compound perfumes such as acetyl cedrene, amyl cinnamaldehyde, allylamyl glycolate, β-ionone, iso E super, isobutyl quinoline, iris oil, irone, ylang ylang oil, undecanal, undecenal, γ-undecalactone, estragole, oakmoss, opoponax resinoid, eugenol, aurantiol, galaxolide, carvacrol, L-carvone, cannon, carrot seed oil, clove oil, methyl cinnamate, geraniol, geranyl nitrile, isobornyl acetate, geranyl acetate, dimethyl benzyl carbinylacetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, tuberose absolute, decanal, decalactone, terpineol, γ-terpinene, triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenyl acetaldehyde, petitgrain oil, hexylcinnamaldehyde, cis-3-hexenol, Peru balsam, vetiver oil, vetiverol, peppermint oil, pepper oil, heliotropine, bergamot oil, benzyl benzoate, borneol, myrrh resinoid, methyl nonyl acetaldehyde, γ-methylionone, menthol, β-ionone, lavender oil, D-limonene, linalool, lyral, lilial, rose absolute, rose oxide, rose oil, and various essential oils can be used.

As the coloring matter, colorant, dye, pigment, for example, one or more kinds selected from certified coloring matters such as Brown No. 201, Black No. 401, Purple Nos. 201 and 401, Blue Nos. 1, 2, 201, 202, 203, 204, 205, 403, and 404, Green Nos. 201, 202, 204, 205, 3, 401, and 402, Red Nos. 102, 104-1, 105-1, 106, 2, 201, 202, 203, 204, 205, 206, 207, 208, 213, 214, 215, 218, 219, 220, 221, 223, 225, 226, 227, 228, 230-1, 230-2, 231, 232, 3, 401, 404, 405, 501, 502, 503, 504, 505, and 506, Orange Nos. 201, 203, 204, 205, 206, 207, 401, 402, and 403, Yellow Nos. 201, 202-1, 202-2, 203, 204, 205, 4, 401, 402, 403-1, 404, 405, 406, 407, and 5; other acidic dyes such as Acid Red 14; basic dyes such as Arianor Sienna Brown, Arianor Madder Red, Arianor Steel Blue, and Arianor Straw Yellow; nitro dyes such as HC Yellow 2, HC Yellow 5, HCRed 3, 4-hydoxypropylamino-3-nitrophenol, N,N'-bis (2-hydroxyethyl)-2-nitro-p-phenylenediamine, HC Blue 2, and Basic Blue 26; disperse dyes; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher, inorganic black pigments such as black iron oxide and lower titanium oxide; inorganic purple pigments such as mango violet and cobalt violet; inorganic green pigments such as chromium oxide, chromium hydroxide, and cobalt titanate; inorganic blue pigments such as ultramarine blue and prussian blue; pearl pigments such as titanium oxide coated mica, titanium oxide coated bismuth oxychloride, titanium oxide coated talc, colored titanium oxide coated mica, bismuth oxychloride, and argentine; metal powder pigments such as an aluminum powder, a copper powder, and gold; surface treatment inorganic and metal powder pigments; organic pigments including zirconium, barium, or aluminum lake such as Red Nos. 201, 202, 204, 205, 220, 226, 228, and 405, Orange Nos. 203, 204, 205, and 401, Blue No. 404, Red Nos. 3, 104, 106, 227, 230, 401, and 505, Orange No. 205, Yellow Nos. 4, 5, 202, and 203, Green No. 3, and Blue No. 1; surface treatment organic pigments; natural coloring matters and dyes such as anthraquinones such as astaxanthin and alizarin, naphthoquinones such as anthocyanidin, β-carotene, carthenal, capsanthin, chalcone, carthamin, quercetin, crocin, chlorophyll, curcumin, cochineal, and shikonin, bixin, flavones, betacyanidin, henna, hemoglobin, lycopene, riboflavin, and rutin; oxidation dye intermediates and couplers such as p-phenylenediamine, toluene-2,5-diamine, o-, m-, or p-aminophenol, m-phenylenediamine, 5-amino-2-methyl phenol, resorcinol, 1-naphthol, 2,6-diaminopyridine, and salts thereof; auto-oxidation type dyes such as indoline; and dihydroxyacetone can be used.

As the water, for example, one or more kinds selected from common water, purified water, and hard water, soft water, natural water, deep ocean water, electrolytic alkaline ion water, electrolytic acidic ion water, ion water, and cluster water can be used.

Examples of the metal-containing compound include lithium compounds such as lithium oxide, butyl lithium, ethyl lithium, lithium bromide, lithium carbide, lithium iodide, lithium nitrate, lithium nitride, lithium sulfide, methyl lithium, lithium sulfate, lithium chloride, lithium hydride, propyl lithium, lithium carbonate, lithium fluoride, lithium niobate, lithium peroxide, lithium hydroxide, lithium molybdate, lithium perchlorate, lithium acetate, lithium formate, lithium tungstate, lithium hypochlorite, lithium acetate dihydrate, lithium iodide dihydrate, llithium hydrogensulfate, lithium acetate dihydrate, lithium bromide monohydrate, lithium sulfate monohydrate, dilithium carbonate, dilithium oxalate, dilithium sulfate, lithium chromate dihydrate, lithium hydroxide monohydrate, lithium citrate tetrahydrate, lithium perchlorate trihydrate, and lithium hexafluorophosphate, and sodium compounds such as sodium amide, sodium oxide, sodium azide, sodium acetate, sodium acetylide, sodium bromide, sodium formate, sodium iodide, sodium lactate, sodium methoxide, sodium nitrate, sodium sulfate, sodium sulfide, sodium borate, metallic sodium, sodium bromate, sodium chloride, sodium hydride, sodium nitrite, sodium sulfite, sodium iodate, sodium phosphate, ecabet sodium, sodium butyrate, heparin sodium, sodium chlorate, sodium citrate, sodium cyanate, sodium cyanide, sodium fluoride, sodium oleate, sodium oxalate, sodium peroxide, sodium silicate, sodium stannate, sodium-t-butoxide, sodium carbonate, suramin sodium, sodium benzoate, sodium chlorite, sodium chromate, sodium hydroxide, sodium selenate, sodium selenide, sodium laurate, disodium inosylate (hereinafter, referred to as IMP), sodium alginate, sodium perborate, sodium persulfate, sodium phosphite, sodium rhodanide, disodium arsenate, sodium selenite, amfenac sodium, tolmetin sodium, sodium pyruvate, saccharin sodium, sodium aluminate, sodium gluconate, sodium polysulfide, sodium stearate, sodium valproate, sodium iopodate, sodium bicarbonate, disodium carbonate, sodium periodate, reviparin sodium, porfimer sodium, carumonam sodium, cefminox sodium, flomoxef sodium, cefalotin sodium, cefazolin sodium, latamoxef sodium, sodium glutamate, sodium molybdate, sodium percarbonate, sodium perchlorate, sodium salicylate, sodium thiosulfate, thiamylal sodium, triclofos sodium, sodium hydrogen carbonate, sodium dichromate, sodium dichromate, sodium fluoroborate, sodium sulfoxylate, disodium phosphate, beraprost sodium, cefotetan sodium, ceftezole sodium, faropenem sodium, trisodium citrate, cefapirin sodium, ampicillin sodium, cefoxitin sodium, cefsulodin sodium, cefuroxime sodium, dantrolene sodium, sodium antimonate, sodium dithionite, sodium propionate, sodium tungstate, sodium pyrobisulfite, trisodium phosphate, sodium triphosphate, dalteparin sodium, parnaparin sodium, danaparoid sodium, cefodizime sodium, carmellose sodium, sodium iothalamate, foscarnet sodium, sodium metabisulfite, cefotaxime sodium, sefpiramide sodium, sodium cyclamate, diclofenac sodium, sodium borohydride, sodium hypochlorite, sodium thiocyanate, thiopental sodium, ticarcillin sodium, fosfomycin sodium, sodium arsenate, dibasic sodium phosphinate, sodium hyaluronate, imitrodast sodium, bucladesine sodium, sodium boron hydroxide, sodium glucuronate, sodium aluminum sulfate, cefmetazole sodium, ceftizoxime sodium, cloxacillin sodium, sodium cyclamate, fluorescein sodium, liothyronine sodium, loxoprofen sodium, piperacillin sodium, sodium hydrosulfite, sodium hypophosphite, sodium naphthionate, sodium permanganate, sodium pyrophosphate, fluvastatin sodium, pravastatin sodium, sodium rabeprazole, sodium methacrylate, disodium 5'-IMP, amobarbital sodium, cefamandole sodium, cefoperazone sodium, sodium ferrocyanide, sodium hydrogensulfide, sodium metavanadate, sodium silicofluoride, sodium xanthate, sodium fluoroacetate, sodium lauryl sulfate, sodium cromoglicate, sodium picosulfate, sodium fluoroacetate, sodium metabisulfite, sodium taurocholate, cerivastatin sodium, sodium acetate trihydrate, ceftriaxone sodium, carbenicillin sodium, cefbuperazone sodium, sodium fumarate, levothyroxine sodium, sodium dehydroacetate, sodium hydrogen sulfite, sulbenicillin sodium, sodium borate decahydrate, sodium metavanadate, sodium metaperiodate, sodium cholate hydrate, sodium sulfate decahydrate, sodium dodecyl sulfate, secobarbital sodium, lobenzarit disodium, sodium deoxycholate, sodium nitroprusside, dicloxacillin sodium, phthalocyanine sodium, sodium thioglycolate, sodium sulfate, sodium dihydronaphthalenidyl, gold sodium thiomalate, 2-biphenylol sodium, sodium cholate n-hydrate, sodium stannate trihydrate, sodium sulfide nonahydrate, calcium disodium edetate, epoprostenol sodium, sodium ferrous citrate, anhydrous sodium carbonate, sodium sulfite heptahydrate, sodium silicoaluminate, caffeine sodium benzoate, disodium hydrogenarsenate, saccharin sodium dihydrate, dibasic sodium phosphate, sodium trichloroacetate, sodium tripolyphosphate, sodium peroxodisulfate, bromfenac sodium hydrate, sodium molybdate dihydrate, basic sodium phosphate, tribasic sodium phosphate, sodium trifluoroacetate, tetrasodium pyrophosphate, sodium chromate tetrahydrate, sodium succinate hexahydrate, sodium dihydrogen citrate, sodium stearyl fumarate, antimony sodium tartrate, sodium cyanoborohydride, sodium dihydrogen phosphate, sodium prasterone sulfate, sodium selenite pentahydrate, dipicrylamine sodium, sodium hexametaphosphate, sodium perborate tetrahydrate, sodium tetrahydroborate, acyl β-alanine sodium, disodium hydrogen phosphate, sodium perchlorate monohydrate, (+)-sodium pantothenate, sodium metasilicate (anhydrous), sodium tetrafluoroborate, sodium sulfanylate dihydrate, sodium cellulose sulfate, pantoprazole sodium hydrate, sodium chondroitin sulfate, disodium methylarsonate, sodium hexafluorosilicate, riboflavin sodium phosphate, dextran sulfate sodium sulfur, trisodium phosphate dodecahydrate, disodium 5'-guanylate, disodium 5'-inosinate, dextran sulfate sodium sulfur 18, sulfobromophthalein sodium, disodium 5'-cytidylate, sodium lauryl sulfonate, betamethasone sodium phosphate, sodium polystyrene sulfonate, prednisolone sodium phosphate, sodium pentachlorophenolate, sodium dichloroisocyanurate, dexamethasone sodium phosphate, estramustine sodium phosphate, prednisolone sodium succinate, sodium hydrogen fumarate, sodium hexacyanoferrate (II), sodium 1-dodecanesulfonate, sodium dihydrogen phosphate dihydrate, carbazochrome sodium sulfonate, hydrocortisone sodium phosphate, sodium 2-hydroxypropanoate, sodium pyrophosphate decahydrate, disodium dihydrogenpyrophosphate, sodium triacetoxyborohydride, hydrocortisone sodium succinate, dibasic sodium phosphate dodecahydrate, colistin sodium methanesulfonate, sodium dodecylbenzene sulfonate, chloramphenicol sodium succinate, sodium 2-hydroxypropionate, sodium (Z)-9-octadecenoate, disodium 2-hydroxysuccinate, sodium cyclohexylsulfamate, flavin adenine dinucleotide sodium, disodium uridine-5'-diphosphate, sodium diphenylamine-4-sulfonate, methylprednisolone sodium succinate, an inosine 5'-monophosphate sodium salt, inosine 5'-phosphate disodium, sodium N-cyclohexylsulfamate, disodium cytidine 5'-phosphate, disodium guanosine-5'-phosphate, an (E)-2-butenedioate hydrogen 1-sodium salt, dexamethasone sodium metasulfobenzoate, sodium 2,4-dichlorophenoxyacetate, sodium (2,4-dichlorophenoxy)acetate, disodium 1,5-naphthalenedisulfonate, pentachlorophenol, sodium salt (for the industry), sodium 4-(2,4-dichloro-m-toluoyl)-1,3-dimethyl-pyrazole-5-olate, sodium 2-methyl-4-chlorophenoxyacetate, sodium (4-chloro-2-methylphenoxy)acetate, sodium [(4-chloro-2-methylphenyl)oxy]acetate, hydrogenated coconut oil fatty acid glycerin sodium sulfate, sodium 5-ethyl-5,8-dihydro-8-oxofuro[3,2-b] [1,8]naphthyridine-7-carboxylate, trisodium 2-hydroxy-1,2,3-propane tricarboxylate, disodium 1-phenylazo-2-naphthol-4',6-disulfonate, sodium 1-(p-sodiosulfophenylazo)-2-hydroxynaphthalene-6-sulfonate, disodium 6-hydroxy-5-[(4-sulfophenyl)azo]-2-naphthalene sulfonate, trisodium 2-hydroxy(1,1'-azobisnaphthalene)-3,4',6-trisulfonate, (+)-N-[(R)-2,4-dihydroxy-3,3-dimethylbutyryl]-β-alanine sodium, disodium 4-[(2,4-dimethylphenyl)azo]-3-hydroxy-2,7-naphthalene disulfonate, sodium (+)-3-[[(R)-2,4-dihydroxy-3,3-dimethyl-butyryl] amino]propionate, and sodium [methyl(2,3-dihydro-1,5-dimethyl-3-oxo-2-phenyl-1H-pyrazol-4-yl)amino]methanesulfo nate.

In addition, examples of the metal-containing organic compound include potassium compounds such as potassium alum, potassium oxide, potassium bromide, potassium iodide, potassium nitrate, potassium sulfate, potassium sulfide, potassium acetate, potassium bromate, potassium chloride, potassium nitrite, potassium sulfite, potassium arsenate, potassium iodate, potassium hydride, potassium t-butoxide, potassium carbonate, potassium chlorate, potassium cyanate, potassium cyanide, potassium fluoride, potassium oxalate, potassium silicate, potassium arsenite, potassium stannate, potassium chromate, potassium hydroxide, potassium phosphate, potassium sorbate, potassium bisulfate, potassium selenate, potassium persulfate, potassium rhodanide, losartan potassium, potassium selenite, potassium tellurite, warfarin potassium, potassium periodate, potassium gluconate, potassium canrenoate, potassium perchlorate, potassium thiosulfate, potassium dichromate, potassium fluoroborate, potassium dichromate, potassium molybdate, potassium pyrosulfite, potassium aspartate, permirolast potassium, potassium hypochlorite, potassium thiocyanate, potassium clavulanate, aluminum potassium sulfate, potassium sulfocyanate, potassium borohydroxide, potassium hypophosphite, potassium metaphosphate, potassium permanganate, potassium pyrophosphate, potassium ferricyanide, potassium ferrocyanide, potassium silicofluoride, potassium hydrogen sulfate, potassium metavanadate, potassium xanthate, potassium hydrogen oxalate, clorazepate dipotassium, potassium selenocyanate, dipotassium oxalate, potassium fluorosilicate, potassium stannate trihydrate, potassium hydrogen carbonate, potassium peroxodisulfate, aluminum potassium sulfate, tetrapotassium pyrophosphate, benzylpenicillin potassium, potassium (methylsulfinyl)methanide, potassium dihydrogen phosphate, dipotassium hydrogenphosphate, dipotassium glycyrrhizate, potassium tetrafluoroborate, potassium guaiacolsulfonate, potassium hexafluorosilicate, aluminum potassium sulfate dodecahydrate, potassium O-ethyldithiocarbonate, potassium hexacyanoferrate (III), phenoxymethyl-penicillin potassium, and potassium (2E, 4E)-2,4-hexadienoate, rubidium compounds such as rubidium oxide, rubidium bromide, rubidium iodide, rubidium sulfate, rubidium chloride, rubidium fluoride, rubidium acetate, rubidium hydrogen sulfate, and rubidium carbonate, cesium compounds such as cesium cyanide, cesium oxide, cesium bromide, cesium iodide, cesium nitrate, cesium sulfate, cesium chloride, cesium carbonate, cesium fluoride, dicesium carbonate, dicesium oxalate, cesium sulfate, dicesium L-tartrate, cesium hydroxide monohydrate, cesium trifluoroacetate, 2,3-dihydroxybutanedioic acid 1-cesium, and 2,3-dihydroxybutanedioic acid hydrogen 1-cesium, beryllium compounds such as beryllium oxide, beryllium bromide, beryllium carbide, beryllium iodide, beryllium nitrate, beryllium nitride, beryllium sulfide, beryllium chloride, beryllium fluoride, beryllium silicate, beryllium distearate, a beryllium sulfate tetrahydrate salt, and an acetylacetone beryllium salt, magnesium compounds such as magnesium oxide, magnesium orotate, aryl magnesium, magnesium acetate, magnesium boride, magnesium bromide, magnesium carbide, magnesium iodide, magnesium nitrate, magnesium nitride, magnesium sulfate, magnesium sulfide, magnesium (powder), magnesium chloride, magnesium carbonate, magnesium chlorate, magnesium citrate, magnesium fluoride, magnesium silicate, magnesium silicide, magnesium hydroxide, magnesium phosphide, magnesium orotate, magnesium (II) nitrate, magnesium stearate, magnesium perchlorate, magnesium myristate, magnesium oxalate, magnesium hydrogen carbonate, magnesium silicofluoride, magnesium fluosilicate, phenyl magnesium bromide, magnesium nitrate hexahydrate, phthalocyanine magnesium, magnesium acetate tetrahydrate, magnesium benzoate trihydrate, calcium magnesium carbonate, magnesium chromate pentahydrate, magnesium hexafluorosilicate, magnesium aluminometasilicate, bismuth magnesium aluminosilicate, and aluminum magnesium hydroxide carbonate hydrate, calcium compounds such as calcium carbide, calcium oxide, calcium bromide, calcium carbide, calcium cyanamide, calcium iodide, calcium lactate, calcium nitrate, calcium nitride, calcium sulfate, calcium sulfide, calcium acetate, calcium chloride, calcium nitrite, calcium sulfite, calcium arsenate, calcium hydride, calcium arsenate, calcium iodate, calcium carbonate, calcium chlorate, calcium fluoride, calcium oxalate, calcium peroxide, calcium silicate, calcium silicide, calcium cyanide, calcium arsenite, heparin calcium, calcium chlorite, calcium chromate, calcium hydroxide, calcium phosphate, calcium phosphide, calcium folinate, calcium gluconate, calcium stearate, calcium aspartate, calcium hypochlorite, fosfomycin calcium, calcium metasilicate, calcium thiocyanate, calcium hypochlorite, calcium pantothenate, calcium permanganate, fenoprofen calcium, calcium chloride dihydrate, calcium magnesium carbonate, polycarbophil calcium, mupirocin calcium hydrate, calcium hydrogen phosphate, calcium glycerophosphate, calcium diiodostearate, precipitated calcium carbonate, calcium disodium edetate, atorvastatin calcium hydrate, (+)-calcium pantothenate, calcium para-aminosalicylate, tocopherol calcium succinate, calcium polystyrene sulfonate, (+)-calcium pantothenate (2:1), calcium aluminoparaaminosalicylate, calcium 3-hydroxy-4-[(4-methyl-2-sulfophenyl)azo]-2-naphthalene-carboxylate, and barium compounds such as barium oxide, barium azide, barium acetate, barium bromide, barium iodide, barium nitrate, barium nitride, barium sulfate, barium sulfide, barium formate, barium lactate, barium chloride, barium sulfite, barium carbonate, barium chlorate, barium fluoride, barium peroxide, barium cyanide, barium chromate, barium hydroxide, barium titanate, barium selenate, barium phosphate, barium (II) nitrate, barium selenite, barium manganate, barium oxalate, barium stearate, barium perchlorate, barium thiosulfate, barium hypochlorite, barium permanganate, barium chloranilate, barium chloride dihydrate, barium diacetate, barium bromide dihydrate, barium dipropionate, barium chlorate monohydrate, barium hydroxide octahydrate, barium thiocyanate dihydrate, barium thiosulfate monohydrate, and acetylacetone barium dihydrate.

As the metal-containing compound, one or more kinds selected from the above-described compounds can be used.

As the unsaturated monomer, for example, one or more kinds selected from hydrophilic unsaturated monomers containing an acid group such as an acrylic acid-based polymer, maleic acid, maleic anhydride, fumaric acid, crotonic acid, itaconic acid, vinyl sulfonic acid, styrene sulfonic acid, 2-(meth)acrylamido-2-methylpropane sulfonic acid, 2-(meth)acryloylethane sulfonic acid, or 2-(meth)acryloylpropane sulfonic acid, or and a salt thereof; nonionic hydrophilic unsaturated monomers such as N-vinylacetamide, N-methyl-N-vinylacetamide, acrylamide, methacrylamide, N-ethyl (meth)acrylamide, N-n-propyl (meth)acrylamide, N-isopropyl (meth)acrylamide, N,N-dimethyl (meth)acrylamide, 2-hydroxyethyl (meth)acrylate, 2-hydroxypropyl (meth)acrylate, methoxypolyethylene glycol (meth)acrylate, polyethylene glycol mono(meth)acrylate, vinyl pyridine, N-vinylpyrrolidone, N-acryloyl piperidine, and N-acryloyl pyrrolidine; and cationic hydrophilic unsaturated monomers such as N,N-dimethylaminoethyl (meth)acrylate, N,N-diethylaminoethyl (meth)acrylate, N,N-dimethylaminopropyl (meth)acrylate, and N,N-dimethylaminopropyl (meth)acrylamide, and quaternary salts thereof, styrene, vinyl chloride, butadiene, isobutene, ethylene, propylene, stearyl (meth)acrylate, and lauryl (meth)acrylate can be used.

As the the polyhydric alcohol, for example, one or more kinds selected from ethylene glycol, propylene glycol, 1,3-butylene glycol, ethylene glycol monobutyl ether, diethylene glycol, triethylene glycol, 1,4-butylene glycol (dihydric alcohol), glycerin, trioxyisobutane (trihydric alcohol), erythrite, pentaerythrite (tetrahydric alcohol), xylit, adonit (pentahydric alcohol), allodulcite, sorbitol, sorbitol liquid, mannitol (hexahydric alcohol), polyglycerin, and dipropylene glycol can be used.

As the the polymer additive, for example, one or more kinds selected from polyvinyl pyrrolidone, a carboxy vinyl polymer which is a cross-linked polyacrylic acid, a vinyl pyrrolidone-ethyl acrylate copolymer, an N-vinyl acetamide-based copolymer such as an N-vinyl acetamide-sodium acrylate copolymer, an N-vinyl acetamide homopolymer, polyvinyl sulfonic acid, an N-vinyl acetamide crosslinked product, polyitaconic acid, hydroxypropyl cellulose, and hydroxypropyl methylcellulose can be used.

As the antiinflammatory analgesic agent, for example, one or more kinds selected from salicylic acid, glycol salicylate, methyl salicylate, 1-menthol, camphor, sulindac, trimethine sodium, naproxen, fenbufen, piroxicam, triamcinolone, hydrocortisone acetate, indomethacin, ketoprofen, acetaminophen, mefenamic acid, flufenamic acid, ibufenac, loxoprofen, tiaprofen, pranoprofen, fenbufen, diclofenac, diclofenac sodium, alclofenac, lornoxicam, pranoprofen, oxyphenbutazone, ibuprofen, felbinac, ketorolac, bermoprofen, nabumetone, naproxen, flurbiprofen, fluocinonide, clobetasol propionate, COX-2 inhibitors (celecoxib, rofecoxib, varecoxib, parecoxib, and valdecoxib) et drag, nimesulide, and meloxicam can be used.

As the antifungal agent, for example, one or more kinds selected from clotrimazole, econazole nitrate, omoconazole nitrate, tioconazole nitrate, ketoconazole nitrate, miconazole nitrate, isoconazole nitrate, tolnaftate, sulconazole nitrate, pyrrolnitrin, pimafucin, undecylenic acid, salicylic acid, siccanin, nystatin, nornaftate, exalamide, phenyliodoundecynoate, thianthol, cyclopiroxolamine, haloprogin, trichomycin, variotin, pentamycin, and amphotericin B can be used.

As the antihistamine agent, for example, one or more kinds selected from antibiotics such as tetracycline hydrochloride, diphenhydramine hydrochloride, chlorpheniramine, diphenyl imidazole, and chloramphenicol, diphenhydramine, chlorpheniramine maleate, diphenhydramine hydrochloride, chlorpheniramine, and diphenylimidazole can be used.

As the hypnotic sedative, for example, one or more kinds selected from phenobarbital, amobarbital, cyclobarbital, lorazepam, and haloperidol can be used.

As the tranquilizer, for example, one or more kinds selected from fluphenazine, thioridazine, diazepam, flunitrazepam, and chlorpromazine can be used.

As the antihypertensive agent, for example, one or more kinds selected from clonidine, clonidine hydrochloride, pindolol, propranolol, propranolol hydrochloride, bufuralol, indenolol, bucumolol, and nifedipine can be used.

As the hypotensive diuretic, for example, one or more kinds selected from hydrothiazide and cyclopenthiazide can be used.

As the antibiotic, for example, one or more kinds selected from penicillin, tetracycline, oxytetracycline, fradiomycin sulfate, erythromycin, and chloramphenicol can be used.

As the anesthetic, for example, one or more kinds selected from lidocaine, benzocaine, ethyl aminobenzoate, and dibucaine can be used.

As the antibacterial substance, for example, one or more kinds selected from benzalkonium chloride, nitrofurazone, nystatin, acetosulfamine, and clotrimazole can be used.

As the antiepileptic agent, for example, one or more kinds selected from nitrazepam, meprobamate, and clonazepam can be used.

As the coronary vasodilator, for example, one or more kinds selected from nitroglycerin, nitroglycol, isosorbide nitrate, erythritol tetranitrate, pentaerythritol tetranitrate, and propatyl nitrate can be used.

As the herbal medicine, for example, one or more kinds selected from cork tree bark, cherry tree, polygala root, zedoary, chamomile, trichosanthes seed, licorice, platycodon root, apricot kernel, bezoar, schisandra fruit, honey locust, bupleurum root, asiasarum root, plantago seed shazenshi, cimicifugae rhizoma, senega, atractylodes lancea rhizome, mulberry root bark, clove, citrus unshiu peel, ipecac, nandina domestica fruit, fritillaria bulb, ophiopogon tuber, pinellia tuber, atractylodes rhizome, henbane, ledebouriella seseloides, and ephedra can be used.

As the auxiliary agent, for example, one or more kinds selected from keratin layer softeners such as ethyl alcohol, isopropyl alcohol, butanol, 1,3-butanediol, propylene glycol, polyethylene glycol # 400, glycerin, crotamiton, benzyl alcohol, phenyl ethyl alcohol, propylene carbonate, hexyl dodecanol, propanol, salicylic acid, allantoin, dimethyl sulfoxide, dimethyl acetamide, dimethyl formamide, diisopropyl adipate, diethyl sebacate, ethyl laurate, lanoline, azone, 1-geranylazacycloheptan-2-one (GACH), dialkylolamide of a fatty acid, salicylic acid, salicylic acid derivatives, urea, and sulfur, moisturizing agents such as pyrrolidone carboxylic acid, surfactants such as propylene glycol monooleate, polyoxyethylene sorbitan monostearate, sorbitan monostearate, and glycerin monostearate, esters such as isopropyl myristate and diethyl sebacate, higher alcohols such as oleyl alcohol, stearyl alcohol, and lauryl alcohol, fatty acids such as stearic acid, hexanoic acid, nonanoic acid, decanoic acid, dodecanoic acid, tetradecanoic acid, hexadecanoic acid, octadecanoic acid, oleic acid, and linoleic acid, terpene-based compounds and surfactants such as menthol, menthone, limonene, pinene, piperitone, terpinene, terpinolene, terpinolol, and carveol, assistants such as allantoin, dimethyl sulfoxide, dimethylacetamide, dimethyl formamide, diisopropyl adipate, diethyl sebacate, ethyl laurate, lanolin, and azone, and, as necessary, cooling agents such as menthol and camphor, oily components such as almond oil, olive oil, camellia oil, persic oil, peppermint oil, sesame oil, soybean oil, mink oil, cottonseed oil, corn oil, safflower oil, coconut oil, eucalyptus oil, castor oil, liquid paraffin, vaseline, squalene, squalane, and lanolin, gelling agents such as a carboxyvinyl polymer, and neutralizers such as diisopropanolamine can be used.

As the wetting agent, for example, one or more kinds selected from glycerine, propylene glycol, sorbitol, 1,3-butylene glycol, dl-pyrrolidone carboxylic acid, and sodium lactate can be used.

As the astringent agent, for example, one or more kinds selected from citric acid, tartaric acid, lactic acid, aluminum chloride, aluminum sulfate, allantoin chlorohydroxy aluminum, allantoin dihydroxy aluminum, aluminum phenolsulfonate, zinc para-phenolsulfonate, zinc sulfate, and aluminum chlorohydroxide can be used.

As the thickening agent, for example, one or more kinds selected from natural polymers such as gum arabic, tragacanth, locust bean gum, guar gum, xanthan gum, karaya gum, agar, starch, carrageenan, alginic acid, alginic acid salts (for example, sodium alginate), propylene glycol alginate, dextran, dextrin, amylose, gelatin, collagen, pullulan, pectin, amylopectin, starch, sodium amylopectin semi-glycolate, chitin, albumin, and casein, semi-synthetic polymers such as polyglutamic acid, polyaspartic acid, methyl cellulose, ethyl cellulose, propyl cellulose, ethyl methyl cellulose, hydroxyethyl cellulose, hydroxypropyl cellulose, hydroxypropyl methyl cellulose, carboxymethyl starch, alkali metal carboxymethyl cellulose, alkali metal cellulose sulfate, a cellulose graft polymer, crosslinked gelatin, cellulose acetate phthalate, a starch-acrylic acid graft polymer, phthalic anhydride-modified gelatin, and succinic acid-modified gelatin, polyvinyl alcohol, polyvinylpyrrolidone, polyvinyl methyl ether, a carboxyvinyl polymer, a vinylpyrrolidone-ethyl acrylate copolymer, a vinylpyrrolidone-styrene copolymer, a vinylpyrrolidone-vinyl acetate copolymer, a vinyl acetate-(meth)acrylic acid copolymer, a polyvinyl acetate-crotonicacid copolymer, N-vinylacetamide-based copolymers such as an N-vinylacetamide-sodium acrylate copolymer, polyvinyl sulfonate, an N-vinylacetamide crosslinked product, polyitaconic acid, polyhydroxyethyl acrylate, polyacrylamide, a styrene-maleic anhydride copolymer, and an acrylamide-acrylic acid copolymer can be used.

As the tackifying material, for example, one or more kinds selected from silicone rubber, polyisoprene rubber, styrene-block copolymer rubber, acrylic rubber, and natural rubber can be used.

As the antipruritic agent, for example, one or more kinds selected from camphor, thymol, menthol, polyoxyethylene lauryl ether, an antihistamine, and ethyl aminobenzoate can be used.

As the keratin layer softening and peeling agent, for example, one or more kinds selected from sulfur, thioxolone, selenium sulfide, salicylic acid, and resorcinol can be used.

As the oily material, for example, one or more kinds selected from almond oil, olive oil, hard oil, camellia oil, castor oil, Japan wax oil, coconut oil, beeswax, spermacetieti, lanolin, carnauba wax, candelilla wax, liquid paraffin, vaseline, microcrystalline wax, ceresine, squalene, lauric acid, myristic acid, palmitic acid, stearic acid, isostearic acid, oleic acid, lauryl alcohol, cetanol, stearyl alcohol, oleyl alcohol, octyl dodecanol, cholesterol, hexyl decanol, white sterol, cetyl lactate, isopropyl myristate, hexyl laurate, myristyl myristate, isopropyl palmitate, octyl myristate dodecanol, butyl stearate, cacao oil, Japan wax, jojoba oil, grape seed oil, avocado oil, mink oil, egg yolk oil, ceresine wax, paraffin wax, behenic acid, isopropyl adipate, octyldodecyl myristate, octyldodecyl oleate, and cholesterol oleate can be used.

As the ultraviolet screening agent, for example, one or more kinds selected from benzophenone-based ultraviolet screening agents such as ASL-24, Cyasorb UV-9, and Uvinul M-40, benzoic acid-based ultraviolet screening agents such as Salol, azole-based ultraviolet screening agents such as Tinuvin P, nitrile-based ultraviolet screening agents such as Uvinul N-35, urea-based ultraviolet screening agents such as Ancour UA, para-amino acid-based ultraviolet screening agents such as Neo Heliopan Give tan F, 2-hydroxy-4-methoxybenzophenone, octyldimethyl para-amino benzoate, and ethylhexyl para-methoxycinnamate, a salicylic acid-based ultraviolet screening agent, a benzofuran-based ultraviolet screening agent, a coumarin-based ultraviolet screening agent, and an azole-based ultraviolet screening agent can be used.

As the antiseptic preservative, for example, one or more kinds selected from acids such as benzoic acid, salicylic acid, dehydroacetic acid, sorbic acid, and boric acid, and salts thereof, phenols such as phenol, chlorocresol, chloroxylenol, isopropyl methyl phenol, resorcinol, orthophenyl phenol, para-oxybenzoic acid ester, phenoxyethanol, thymol, hinokitiol, and thioxolone, halogenated bisphenols such as hexachlorophene and 2,4,4'-trichloro-2'-hydroxydiphenyl ether, amide compounds such as trichlorocarbanilide, halocarban, and undecylenic acid monoethanolamide, quaternary ammonium compounds such as benzalkonium chloride, alkyl isoquinolinium bromide, benzethonium chloride, and cetylpyridinium chloride, and amphoteric surfactants such as rauryl di(aminoethyl)glycine, a 2-pyridine thiol-1-oxide zinc salt, gluconic acid, chlorhexidine, thiram, N-trichloromethyl thio-4-cyclohexene-1,2-dicarboximide, and chlorobutanol can be used.

As the antioxidant, for example, one or more kinds selected from methyl L-ascorbate, ethyl L-ascorbate, propyl L-ascorbate, butyl L-ascorbate, pentyl L-ascorbate, L-ascorbyl 2-palmitate, L-ascorbyl-2-isopalmitate, L-ascorbyl-2-stearate, L-ascorbyl-2-isostearate, L-ascorbyl-6-palmitate, L-ascorbyl-6-isopalmitate, L-ascorbyl-6-stearate, L-ascorbyl-6-isostearate, L-ascorbyl-2,6-dipalmitate, L-ascorbyl-2,6-diisopalmitate, L-ascorbyl-2,6-distearate, L-ascorbyl-2,6-diisostearate, L-ascorbyl-2-glucoside-6-palmitate, L-ascorbyl-2-glucoside-6-isopalmitate, L-ascorbyl-2-glucoside-6-stearate, L-ascorbyl-2-glucoside-6-isostearate, L-ascorbyl-2-glucoside-6-tocopheryl, L-ascorbyl-2-sulfate-6-palmitate, L-ascorbyl-2-sulfate-6-isopalmitate, L-ascorbyl-2-sulfate-6-stearate, L-ascorbyl-2-sulfate-6-isostearate, L-ascorbyl-2-sulfate-6-tocopheryl, L-ascorbyl-2-maleate-6-palmitate, L-ascorbyl-2-maleate-6-isopalmitate, L-ascorbyl-2-maleate-6-stearate, L-ascorbyl-2-maleate-6-isostearate, L-ascorbyl-2-maleate-6-tocopheryl, L-ascorbyl-2-phosphate-6-palmitate, L-ascorbyl-2-phosphate-6-isopalmitate, L-ascorbyl-2-phosphate-6-stearate, L-ascorbyl-2-phosphate-6-isostearate, L-ascorbyl-2-phosphate-6-tocopheryl, palmitoyl 2-0-L-ascorbic aicid, isopalmitoyl 2-0-L-ascorbyl phosphate, stearyl 2-0-L-ascorbyl phosphate, isostearyl 2-0-L-ascorbyl phosphate, tocopheryl 2-0-L-ascorbyl phosphate, retinoyl 2-0-L-ascorbyl phosphate, tocopheryl phosphate, tocotrienol phosphate, tocopheryl glucoside, tocopheryl sulfate, tocopheryl dimethyl glycine, retinoyl phosphate, retinoyl glucoside, retinoyl sulfate, and retinoyl dimethyl glycine can be used.

As the liquid matrix, for example, one or more kinds selected from glycerin, polyhydric alcohol, ethanol, propanol, isopropanol, a gelling agent, glycerin, methyl laurate, lauryl alcohol, glycerol monolaurate, oleic acid, oleyl alcohol, glycerol monooleate, glycerol dioleate, glycerol trioleate, nicotinic acid, 2-pyrazine carboxylic acid, sinapic acid (3,5-dimethoxy-4-hydroxycinnamic acid), 2,5-dihydroxybenzoic acid, 5-methoxysalicylic acid, α-cyano-4-hydroxycinnamic acid, 3-hydroxy picolinic acid, diaminonaphthalene, 2-(4-hydroxyphenylazo)benzoic acid, dithranol, succinic acid, 5-(trifluoromethyl)uracil, propylene glycol, dipropylene glycol, polypropylene glycol, glycerin, diglycerin, polyglycerin, ethylene glycol, diethylene glycol, polyethylene glycol, 1,3-butanediol, sorbitol, maltitol, erythritol, pentaerythritol, glucose, saccharose, maltose, xylose, and trehalose can be used.

As the fat-soluble material, for example, one or more kinds selected from ascorbyl isopalmitate, ascorbyl-2-phosphate-6-palmitate, ascorbyl-2-phosphate-6-isopalmitate, ascorbyl tetrahexyldecanoate, ascorbyl-6-palmitate, ascorbyl-2,6-dipalmitate, ascorbyl-6-stearate, retinol, retinoic acid, retinol palmitate, tocopheryl retinoate, retinyl palmitate, retinyl isopalmitate, retinyl stearate, retinol stearate, astaxanthin, lutein, lycopene, β-carotene, α-carotene, α-tocopherol, β-tocopherol, γ-tocopherol, dimethyl glycine tocopherol, ubiquinone, coenzyme A, tocotrienol, mineral oil, liquid paraffin, squalane, isopropyl palmitate, isopropyl myristate, isooctyl myristate, isotridecyl myristate, octadecyl myristate, octyldodecyl myristate, isostearyl cholesteryl ester, triglyceride 2-ethylhexanoate, cetyl 2-ethyl hexanoate, sunflower oil, olive oil, jojoba oil, camellia oil, grape seed oil, avocado oil, macadamia nut oil, almond oil, rice germ oil, clove oil, orange oil, orange peel oil, pycnogenol, coenzyme A, iris extract oil, ashitaba extract oil, thujopsis dolobrata extract oil, asparagus extract oil, avocado extract oil, sweet hydrangea leaf extract oil, almond extract oil, althea extract oil, arnica extract oil, aloe extract oil, apricot extract oil, apricot kernel extract oil, ginkgo extract oil, inchikow extract oil, fennel extract oil, turmeric extract oil, oolong tea extract oil, uva ursi extract oil, rose fruit extract oil, echinacea leaf extract oil, isodon japonicus extract oil, scutellaria root extract oil, phellodendron bark extract oil, coptis japonica extract oil, barley extract oil, panax ginseng extract oil, hypericum extract oil, lamium album extract oil, ononis spinosa extract oil, Netherland mustard extract oil, orange extract oil, a dried seawater product, seaweed extract oil, persimmon leaf extract oil, pyracantha fortuneana fruit extract oil, hydrolyzed elastin, a hydrolyzed wheat powder, hydrolyzed silk, pueraria root extract oil, chamomilla extract oil, carrot extract oil, capillary artemisia extract oil, wild oat extract oil, hibiscus sabdariffa extract oil, licorice extract oil, oil-soluble licorice extract oil, kiwi extract oil, kiou apple extract oil, Jew's ear mushroom extract oil, cinchona bark extract oil, cucumber extract oil, paulownia tomentosa leaf extract oil, guanosine, guava extract oil, sophora root extract oil, gardenia extract oil, kuma bamboo grass extract oil, sophora flavescens extract oil, walnut extract oil, chestnut extract oil, grapefruit extract oil, clematis extract oil, black rice extract oil, fat-soluble brown sugar extract, fat-soluble black vinegar extract, chlorella extract oil, mulberry extract oil, gentian extract oil, geranium thumbergii extract oil, black tea extract oil, yeast extract oil, magnolia bark extract oil, coffee extract oil, burdock extract oil, rice extract oil, fermented rice extract oil, fermented rice bran extract oil, rice germ oil, comfrey extract oil, collagen, cowb extract oil, saisin extract oil, bupleurum root extract oil, umbilical cord extract liquid, saffron extract oil, salvia extract oil, saponaria officinalis extract oil, sasa extract oil, hawthorn extract oil, sansha extract oil, Japanese pepper extract oil, shiitake mushroom extract oil, rehmannia root extract oil, lithospermum root extract oil, perilla extract oil, linden extract oil, meadowsweet extract oil, jatoba extract oil, peony extract oil, ginger extract oil, calamus root extract oil, white birch extract oil, white Jew's ear mushroom extract oil, horsetail extract oil, stevia extract oil, a stevia-fermented product, Chinese tamarisk extract oil, English ivy extract oil, whitethorn extract oil, Sambucus nigra extract oil, yarrow extract oil, peppermint extract oil, sage extract oil, mallow extract oil, cnidium rhizome extract oil, Japanese green gentian extract oil, mulberry bark extract oil, rhubarb extract oil, soybean extract oil, jujube extract oil, thyme extract oil, dandelion extract oil, lichens extract oil, tea extract oil, clove extract oil, imperata extract oil, citrus unshiu peel extract oil, tea tree oil, Chinese blackberry extract oil, capsicum extract oil, Japanese angelica root extract oil, calendula officinalis extract oil, peach kernel extract oil, spruce extract oil, houttuynia extract oil, tomato extract oil, natto extract oil, carrot extract oil, garlic extract oil, eglantine extract oil, hibiscus extract oil, ophiopogon extract oil, lotus extract oil, parsley extract oil, birch extract oil, honey, hamamelis extract oil, parietaria extract oil, isodon japonicus extract oil, bisabolol, cypress extract oil, Lactobacillus bifidus extract oil, loquat extract oil, coltsfoot extract oil, petasites japonicus extract oil, hoelen extract oil, butcher bloom extract oil, grape extract oil, grape seed extract oil, propolis, loofah extract oil, safflower extract oil, peppermint extract oil, linden extract oil, tree peony extract oil, hop extract oil, rosa rugosa extract oil, pine extract oil, horse chestnut extract oil, skunk cabbage extract oil, soapberry extract oil, melissa extract oil, mozuku extract oil, peach extract oil, cornflower extract oil, eucalyptus oil extract, saxifrage extract oil, yuzu extract oil, lily extract oil, coix seed extract oil, wormwood extract oil, lavender extract oil, green tea extract oil, egg shell membrane extract oil, apple extract oil, rooibos tea extract oil, litchi extract oil, lettuce extract oil, lemon extract oil, weeping forsythia extract oil, astragalus sinicus extract oil, rose extract oil, rosemary extract oil, roman chamomile extract oil, royal jelly extract oil, burnet extract oil, octamethyl trisiloxane, decamethyl tetrasiloxane, methyl polysiloxane, dimethyl silicone oils such as highly polymerized methyl polysiloxane; cyclic silicone oils such as octamethyl cyclotetrasiloxane, decamethyl cyclopentasiloxane, dodecamethyl cyclohexasiloxane, methyl cyclopolysiloxane, and methyl polycyclosiloxane; and methyl phenyl silicone oils such as polyether-modified silicone oil and methyl phenyl polysiloxane can be used.

As the polymer carboxylate, for example, one or more kinds selected from acrylic resins such as polyacrylic acid, an acrylic acid-acrylonitrile copolymer, a potassium acrylate-acrylonitrile copolymer, a vinyl acetate-acrylic acid ester copolymer, and an acrylic acid-acrylic acid alkyl ester copolymer, styrene acrylic resin such as a styrene-acrylic acid copolymer, a styrene-methacrylic acid copolymer, a styrene-methacrylic acid-acrylic acid alkyl ester copolymer, a styrene-α-methyl styrene-acrylic acid copolymer, and a styrene-α-methyl styrene-acrylic acid-acrylic acid alkyl ester copolymer; a styrene-maleic acid copolymer and a styrene-maleic anhydride copolymer; vinyl acetate resins such as a vinyl naphthalene-acrylic acid copolymer, a vinyl acetate-ethylene copolymer, a vinyl acetate-fatty acid vinyl ethylene copolymer, a vinyl acetate-maleic acid ester copolymer, a vinyl acetate-crotonic acid copolymer, and a vinyl acetate-acrylic acid copolymer; and a salt of any polyester or cellulose polymer can be used.

As the additive, for example, one or more kinds selected from hydrocarbons, waxes, fatty acids, higher alcohols, ester oils, silicone oils, fluorine-based oils, that is, hydrocarbons such as ozokerite, squalane, squalene, ceresine, paraffin, paraffin wax, liquid paraffin, pristane, polyisobutylene, microcrystalline wax, and vaseline, waxes such as beeswax, carnauba wax, candelilla wax, and spermacetieti, animal oils such as beef tallow, neats-foot oil, beef bone fat, hard beef tallow oil, hard oil, turtle oil, lard, horse fat, mink oil, cod-liver oil, and egg yolk oil, lanolin derivatives such as lanolin, liquid lanolin, reduced lanolin, lanolin alcohol, a hard lanolin, lanolin acetate, fatty acid lanolin isopropyl, phospholipid, phosphatidylcholine, POE lanolin alcohol ether, POE lanolin alcohol acetate, fatty acid lanolin polyethylene glycol, and POE hydrogenated lanolin alcohol ether, fatty acids such as lauric acid, myristic acid, palmitic acid, stearic acid, behenic acid, undecylenic acid, oleic acid, linoleic acid, arachidonic acid, eicosapentaenoic acid (EPA), docosahexaenoic acid (DHA), isostearic acid, and 12-hydroxy stearic acid, higher alcohols such as lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol-2-decyltetradecinol, cholesterol, phytosterol, sitosterol, lanosterol, POE cholesterol ether, and monostearyl glycerin ether (batyl alcohol), ester oils such as adipic acid diisobutyl ester, adipic acid 2-hexyl decyl ester, adipic acid di-2-heptylundecyl ester, monoisostearic acid N-alkyl glycol ester, isostearic acid isocetyl ester, triisostearic acid trimethylol propane ester, di-2-ethylhexanoic acid ethylene glycol 2-ethylhexanoic acid cetyl ester, tri-2-ethylhexanoic acid trimethylol propane ester, tetra-2-ethylhexanoic acid pentaerythritol ester, octanoic acid cetyl ester, octyldodecyl gum ester, oleic acid oleyl ester, oleic acid octyldodecyl ester, oleic acid decyl ester, dicapric acid neopentyl glycol ester, cirtic acid triethyl ester, succinic acid 2-ethylhexyl ester, acetic acid amyl ester, acetic acid ethyl ester, acetic acid butyl ester, stearic acid isocetyl ester, stearic acid butyl ester, sebacic acid diisopropyl ester, sebacic acid di-2-ethylhexyl ester, lactic acid cetyl ester, lactic acid myristyl ester, palmitic acid isopropyl ester, palmitic acid 2-ethylhexyl ester, palmitic acid 2-hexyldecyl ester, palmitic acid 2-heptylundecyl ester, 12-hydroxy stearic acid cholesteryl ester, dipentaerythritol fatty acid ester, myristic acid isopropyl ester, myristic acid octyldodecyl ester, myristic acid 2-hexyldecyl ester, myristic acid myristyl ester, dimethyl octanoic acid hexyldecyl ester, lauric acid ethyl ester, lauric acid hexyl ester, N-lauroyl-L-glutamic acid 2-octyldodecyl ester, and malic acid diisostearyl ester, glyceride oils such as acetoglyceride, triisooctanoic acid glyceride, triisostearic acid glyceride, triisopalmitic acid glyceride, tri-2-ethyl hexanoic acid glyceride, monostearic acid glyceride, di-2-heptyl undecanoic acid glyceride, and trimyristic acid glyceride, higher alkoxy-modified silicones such as dimethyl polysiloxane, methylphenyl polysiloxane, methylhydrogen polysiloxane, octamethyl cyclotetrasiloxane, decamethyl cyclopentasiloxane, dodecamethyl cyclohexasiloxane, tetramethyl tetrahydrogen cyclotetrasiloxane, and stearoxy silicone, silicone oils such as higher fatty acid-modified silicone, a silicone resin, silicon rubber, and silicone resin, and fluorine-based oils such as perfluoropolyether, perfluorodecalin, and perfluorooctane can be used.

As the metal soap, for example, one or more kinds selected from aluminum 12-hydroxystearate, zinc stearate, aluminum stearate, calcium stearate, magnesium stearate, zinc myristate, magnesium myristate, zinc cetyl phosphate, calcium cetyl phosphate, sodium zinc cetyl phosphate, zinc laurate, and zinc undecylenate can be used.

The composition for improving a skin barrier function of the embodiment may contain 0.01% to 50% by mass of the above skin barrier function-improving agent. In other words, the composition for improving a skin barrier function of the embodiment may contain 0.01% to 50% by mass of the above inositol derivative.

In the composition for improving a skin barrier function of the embodiment, the amount of the skin barrier function-improving agent described above may be, for example, 0.01% to 20% by mass, and for example, may be 0.1% to 10% by mass.

When the amount of the skin barrier function-improving agent is within the above range, it is suitable to exert the effect of the composition for improving a skin barrier function on promoting the production of TRPV4, claudins, and occludin, which leads to the effective improvement of the moisture-retaining function of the skin, formulation is easy, and it is possible to easily maintain stability as an agent.

The administration method of the skin barrier function-improving agent or the composition for improving a skin barrier function is not particularly limited, and may be suitably determined depending on the conditions, weight, age, sex, and the like of a patient. For example, a tablet, a coated tablet, a pill, a powder, a granule, a capsule, a solution, a suspension, an emulsion, or the like is administered orally. Injections are intravenously administered alone or in combination with an ordinary replacement solution such as glucose, amino acid, or the like, and, as necessary, injections are administered intraarterially, intramuscularly, intradermally, subcutaneously, or intraperitoneally. Suppositories are administered intrarectally. The skin external agent is applied to an affected part, patched, or sprayed.

Since the dose of the skin barrier function-improving agent or the composition for improving a skin barrier function varies depending on the conditions, weight, age, sex, and the like of a patient, it cannot be unconditionally determined; however, in the case of oral administration, for example, 0.01 to 500 mg of the active ingredient per unit dose form (inositol derivative) may be administered. In addition, in the case of injections, for example, an active ingredient of 0.02 to 250 mg of the active ingredient per unit dose form may be administered. In addition, in the case of suppositories, for example, an active ingredient of 0.01 to 500 mg of the active ingredient per unit dose form may be administered. In addition, in the case of skin external agents, for example, an active ingredient of 0.15 to 500 mg of the active ingredient per unit dose form may be administered.

Since the dose of the skin barrier function-improving agent or the composition for improving a skin barrier function per day varies depending on the conditions, weight, age, sex, and the like of a patient, it cannot be unconditionally determined; however, for an adult, for example, 0.05 to 500 mg of the active ingredient per day may be administered once per day or 1 to 3 times per day by dividing the amount of the active ingredient.

In one embodiment, the present invention provides a method for improving a skin barrier function, the method including a step of administrating an inositol derivative to mammals. As an inositol derivative, the same compounds as described above can be used. In the method of the embodiment, an inositol derivative may be a composition including an inositol derivative and a pharmacologically acceptable carrier.

In one embodiment, the present invention provides a method for promoting the production of TRPV4, claudins, or occludin, the method including a step of administrating an inositol derivative to mammals. As an inositol derivative, the same compounds as described above can be used. In the method of the embodiment, an inositol derivative may be a composition including an inositol derivative and a pharmacologically acceptable carrier.

In one embodiment, the present invention provides a method for enhancing a moisture-retaining function of the skin, the method including a step of administrating an inositol derivative to mammals. As an inositol derivative, the same compounds as described above can be used. In the method of the embodiment, an inositol derivative may be a composition including an inositol derivative and a pharmacologically acceptable carrier.

In one embodiment, the present invention provides a method for treating xerosis cutis, the method including a step of administrating an inositol derivative to mammals. As an inositol derivative, the same compounds as described above can be used. In the method of the embodiment, an inositol derivative may be a composition including an inositol derivative and a pharmacologically acceptable carrier.

In one embodiment, the present invention provides an inositol derivative for improving a skin barrier function. As an inositol derivative, the same compounds as described above can be used in the embodiment. The inositol derivative of the embodiment may be a composition including an inositol derivative and a pharmacologically acceptable carrier.

In one embodiment, the present invention provides an inositol derivative for promoting the production of TRPV4, claudins, or occludin. As an inositol derivative, the same compounds as described above can be used in the embodiment. The inositol derivative of the embodiment may be a composition including an inositol derivative and a pharmacologically acceptable carrier.

In one embodiment, the present invention provides an inositol derivative for enhancing a moisture-retaining function of the skin. As an inositol derivative, the same compounds as described above can be used in the embodiment. The inositol derivative of the embodiment may be a composition including an inositol derivative and a pharmacologically acceptable carrier.

In one embodiment, the present invention provides an inositol derivative for treating xerosis cutis. As an inositol derivative, the same compounds as described above can be used in the embodiment. The inositol derivative of the embodiment may be a composition including an inositol derivative and a pharmacologically acceptable carrier.

In one embodiment, the present invention provides the use of an inositol derivative for producing a skin barrier function-improving agent. As an inositol derivative, the same compounds as described above can be used in the embodiment. The inositol derivative of the embodiment may be a composition including an inositol derivative and a pharmacologically acceptable carrier.

In one embodiment, the present invention provides the use of an inositol derivative for producing a promoter for TRPV4 production, a promoter for claudins production, or a promoter for occludin production. As an inositol derivative, the same compounds as described above can be used in the embodiment. The inositol derivative of the embodiment may be a composition including an inositol derivative and a pharmacologically acceptable carrier.

In one embodiment, the present invention provides the use of an inositol derivative for producing an enhancer for a moisture-retaining function of the skin. As an inositol derivative, the same compounds as described above can be used in the embodiment. The inositol derivative of the embodiment may be a composition including an inositol derivative and a pharmacologically acceptable carrier.

In one embodiment, the present invention provides the use of an inositol derivative for producing a therapeutic agent for xerosis cutis. As an inositol derivative, the same compounds as described above can be used in the embodiment. The inositol derivative of the embodiment may be a composition including an inositol derivative and a pharmacologically acceptable carrier.

### Examples

Hereinafter, the present invention will be described based on Experimental Examples, but the present invention is not limited to the following Experimental Examples.

### [Experimental Example 1]

### (Promoting Effect on TRPV4 Gene Expression)

Human epidermal cells (SVHK cells) which were established from introduction of SV40 gene into human epidermal cells, were seeded on a plastic petri dish at a seeding density of 10,000 cells/cm² and cultured for 24 hours in Dulbecco's Modified Eagle's Medium (DMEM, manufactured by Sigma-Aldrich Co. LLC.) containing 10% fetal bovine serum.

Next, to the medium of SVHK cells, an inositol derivative (composition thereof will be described below) dissolved in purified water was added to the final concentration of 1 µM, and culturing was further performed for 48 hours. As for a control, in place of 1 µM inositol derivative, the same amount of purified water was added to SVHK cells, and the cells were cultured in the same manner as above. In addition, for comparison, 1 µM myo-inositol dissolved in purified water was added to SVHK cells in place of the inositol derivative, and the cells were cultured in the same manner as above. The inositol derivative used was analyzed by using liquid chromatography-mass spectrometry (LC-MS), and the results are as follows. The ratio of molecules where the number of glucose in the glucose chain which is bonded to myo-inositol is one was 12% by mass, the ratio of molecules where the number of glucose in the glucose chain which is bonded to myo-inositol is two was 30% by mass, the ratio of molecules where the number of glucose in the glucose chain which is bonded to myo-inositol is three was 9% by mass, the ratio of molecules where the number of glucose in the glucose chain which is bonded to myo-inositol is four was 12% by mass, the ratio of molecules where the number of glucose in the glucose chain which is bonded to myo-inositol is five was 2% by mass.

Next, all RNA was extracted from cells of each group and cDNA was synthesized. Next, quantitative real-time PCR was performed using this cDNA as a template, and the expression levels of TRPV4 gene in SVHK cells of each group were quantified. In addition, the expression level of glyceraldehyde-3-phosphate dehydrogenase (GAPDH) which is a housekeeping gene in which variation is not observed in the expression by adding a compound, as an internal standard gene, was quantified, and based on the expression levels of GAPDH gene in SVHK cells of each group, the expression levels of TRPV4 gene were standardized. As a primer for GAPDH gene amplification, primer GAPDH (ID: HA067812) manufactured by TAKARA BIO INC. was used. In addition, as a primer for TRPV4 gene amplification, primer TRPV4 (ID: HA186338) manufactured by TAKARA BIO INC. was used.

The expression levels of the standardized TRPV4 gene are shown in Table 1. The inositol derivative exhibited remarkable promoting effect on TRPV4 gene expression as compared to the group added with purified water as the control. In addition, the promoting effect of the inositol derivative on TRPV4 gene expression was remarkably higher than the promoting effect of the myo-inositol on TRPV4 gene expression.

**[Table 1]**

| Sample | Expression levels of TRPV4 gene (relative value) |
|---|---|
| Purified water | 1.00 |
| Myo-inositol | 0.84 |
| Inositol derivative | 8.11 |

### [Experimental Example 2]

### (Promoting Effect on TJ-Related Gene Expression)

Human epidermal cells (SVHK cells) which were established from introduction of SV40 gene into human epidermal cells, were seeded on a plastic petri dish at a seeding density of 10,000 cells/cm² and cultured for 24 hours in Dulbecco's Modified Eagle's Medium (DMEM, manufactured by Sigma-Aldrich Co. LLC.) containing 10% fetal bovine serum.

Next, to the medium of SVHK cells, 1 µM inositol derivative (composition thereof will be described below) dissolved in purified water was added to the final concentration of 1 µM, and culturing was further performed for 48 hours. As for a control, in place of the inositol derivative, the same amount of purified water was added to SVHK cells, and the cells were cultured in the same manner as above. In addition, for comparison, 1 µM myo-inositol dissolved in purified water was added to SVHK cells in place of the inositol derivative, and the cells were cultured in the same manner as above. The inositol derivative used was analyzed by using liquid chromatography-mass spectrometry (LC-MS), and the results are as follows. The ratio of molecules where the number of glucose in the glucose chain which is bonded to myo-inositol is one was 12% by mass, the ratio of molecules where the number of glucose in the glucose chain which is bonded to myo-inositol is two was 30% by mass, the ratio of molecules where the number of glucose in the glucose chain which is bonded to myo-inositol is three was 9% by mass, the ratio of molecules where the number of glucose in the glucose chain which is bonded to myo-inositol is four was 12% by mass, the ratio of molecules where the number of glucose in the glucose chain which is bonded to myo-inositol is five was 2% by mass.

Next, all RNA was extracted from cells of each group and cDNA was synthesized. Next, quantitative real-time PCR was performed using this cDNA as a template, and the expression levels of claudin-1 (CLDN1) gene and occludin (OCL) gene in SVHK cells of each group were quantified.

In addition, the expression level of glyceraldehyde-3-phosphate dehydrogenase (GAPDH) which is a housekeeping gene in which variation is not observed in the expression by adding a compound, as an internal standard gene, was quantified, and based on the expression levels of GAPDH gene in SVHK cells of each group, the expression levels of CLDN1 gene and OCL gene were standardized. As a primer for GAPDH gene amplification, primer GAPDH (ID: HA067812) manufactured by TAKARABIO INC. was used. In addition, as a primer for CLDN1 gene amplification, primer CLDN1 (ID: HA103348) manufactured by TAKARA BIO INC. was used. Furthermore, as a primer for OCL gene amplification, primer OCL (ID: HA036753) manufactured by TAKARA BIO INC. was used.

The expression levels of the standardized CLDN1 gene and OCL gene are shown in Table 2. The inositol derivative exhibited remarkable promoting effect on CLDN1 gene and OCL gene expressions as compared to the group added with purified water as the control. In addition, the promoting effect of the inositol derivative on CLDN1 gene and OCL gene expressions was remarkably higher than the promoting effect of the myo-inositol on CLDN1 gene and OCL gene expressions.

**[Table 2]**

| Sample | Expression levels of gene (relative value) | |
|---|---|---|
| | CLDN1 | OCL |
| Purified water | 1.00 | 1.00 |
| Myo-inositol | 1.57 | 0.94 |
| Inositol derivative | 61.28 | 7.45 |

### [Experimental Example 3]

### (Test for Evaluating TJ-forming Ability)

Skin Model (type "EP1-200" manufactured by KURABO INDUSTRIES LTD.) was cultured before 24 hours at 37°C under 5% CO₂. Next, an inositol derivative (composition thereof will be described below) dissolved in purified water in each concentration of 0.1, 0.5, and 1 w/v% was added to the surface of Skin Model, and culturing was further performed for 24 hours.

Next, the inositol derivative was removed, Skin Model was washed three times with a phosphate buffer solution, and transepidermal water loss (TEWL) thereof was measured. For the measurement of TEWL, a cyclone-type moisture-transpiration meter (manufactured by ASAHIBIOMED) was used. The inositol derivative used was analyzed by using liquid chromatography-mass spectrometry (LC-MS), and the results are as follows. The ratio of molecules where the number of glucose in the glucose chain which is bonded to myo-inositol is one was 12% by mass, the ratio of molecules where the number of glucose in the glucose chain which is bonded to myo-inositol is two was 30% by mass, the ratio of molecules where the number of glucose in the glucose chain which is bonded to myo-inositol is three was 9% by mass, the ratio of molecules where the number of glucose in the glucose chain which is bonded to myo-inositol is four was 12% by mass, the ratio of molecules where the number of glucose in the glucose chain which is bonded to myo-inositol is five was 2% by mass.

As for a control, prepared were a group added with nothing in place of the inositol derivative and a group added with the same amount of purified water in place of the inositol derivative, and TEWL was measured in the same manner as above. In addition, for comparison, prepared was a group in which myo-inositol dissolved in purified water in a concentration of 1 w/v% was added in place of the inositol derivative, and TEWL was measured in the same manner as above.

The results of TEWL measurement are shown in Table 3. It was confirmed that in the group added with the inositol derivative, TJ formation was promoted, and therefore a moisturizing ability (moisture-retaining function) was enhanced.

**[Table 3]**

| Sample | TEWL [g/(h·m²)] |
|---|---|
| Non-applied | 24.3±1.5 |
| Purified water | 22.7±3.4 |
| 1% myo-inositol | 30.7±2.9 |
| 0.1% inositol derivative | 29.3±3.2 |
| 0.5% inositol derivative | 31.6±1.4 |
| 1% inositol derivative | 33.6±2.1 |

After measuring TEWL, to the medium in contact with the lower surface of Skin Model, fluorescent labelled biotin was added to the final concentration of 0.1 mg/mL as a tracer, and culturing was performed for one hour at 37°C under 5% CO₂. The tracer spread from the lower surface of Skin Model to the upper part where a stratum corneum is present. In a case where tight junctions (TJ) were formed and thus a barrier was present, the spread of the tracer stopped at the lower part of TJs and the tracer spread no farther. On the other hand, in a case where a barrier was damaged, the tracer spread from a stratum granulosum of the epidermis to immediately below the stratum corneum. Therefore, whether Skin Model has a barrier function or not can be determined with a microscope. Next, Skin Model was collected and paraffin-embedded, and a specimen of tissue slices was prepared by using a slice preparation device.

Next, the specimen was reacted with Rabbit Anti-Occludin antibody (produced by Abcam plc.), further reacted with FITC-labelled Donkey Anti-Rabbit antibody (produced by Abcam plc.), and subjected to immunostaining. Next, FITC fluorescence of the specimen subjected to immunostaining was observed with a fluorescence microscope. In fluorescence microscopy, a fluorescence filter having an excitation wavelength of 488 nm and a fluorescence wavelength of 543 nm was used.

The obtained fluorescence microscopy image was binarized by using software (product name "Photoshop" produced by Adobe Systems Incorporated.), and fluorescence intensities were digitalized. Values of the digitalized fluorescence intensities were evaluated as a barrier ability obtained by the formation of tight junctions. The fluorescence intensity values of each group are relative values with respect to a measured value of a non-applied group. The results are shown in Table 4. It became clear that the expression levels of occludin in terms of protein levels were increased by adding the inositol derivative. The results indicate that a TJ-forming ability was enhanced by adding the inositol derivative.

**[Table 4]**

| Sample | Fluorescence intensity (relative value) |
|---|---|
| Non-applied | 1.00 |
| Purified water | 1.12 |
| 1% myo-inositol | 3.32 |
| 0.1% inositol derivative | 3.22 |
| 0.5% inositol derivative | 4.34 |
| 1% inositol derivative | 5.98 |

### Industrial Applicability

According to the present invention, it is possible to provide a skin barrier function-improving agent and a composition for improving a skin barrier function which act on both TRPV4 and TJs, and effectively enhance a moisture-retaining function of the skin.

## Claims

1. A skin barrier function-improving agent comprising an inositol derivative as an active ingredient in which inositol and saccharide are bonded.

2. The skin barrier function-improving agent according to Claim 1, wherein the saccharide is monosaccharide or oligosaccharide.

3. The skin barrier function-improving agent according to Claim 2, wherein the monosaccharide is glucose.

4. The skin barrier function-improving agent according to Claim 2, wherein the oligosaccharide contains glucose as a structural unit.

5. The skin barrier function-improving agent according to any one of Claims 1 to 4, wherein the inositol is myo-inositol.

6. The skin barrier function-improving agent according to any one of Claims 1 to 5, which promotes the production of TRPV4.

7. The skin barrier function-improving agent according to any one of Claims 1 to 6, which promotes the production of claudins.

8. The skin barrier function-improving agent according to any one of Claims 1 to 7, which promotes the production of occludin.

9. A composition for improving a skin barrier function comprising:
the skin barrier function-improving agent according to any one of Claims 1 to 8; and
a pharmacologically acceptable carrier.

10. The composition for improving a skin barrier function according to Claim 9, wherein the amount of the skin barrier function-improving agent is 0.01 to 50% by mass.

11. The composition for improving a skin barrier function according to Claim 9 or 10, which is a skin external agent.

12. The composition for improving a skin barrier function according to any one of Claims 9 to 11, which is a cosmetic.
